# EUROPÄISCHE PATENTSCHRIFT

(11) **EP 2 757 182 B1**
(45) Veröffentlichungstag und Bekanntmachung des Hinweises auf die Patenterteilung: **08.08.2018**
(21) Anmeldenummer: 14150896.0
(22) Anmeldetag: 13.01.2014
(51) Int. Cl.: C25F 3/00, A61L 27/00

(54) **VERFAHREN ZUR HERSTELLUNG EINES MIKROSTRUKTURIERTEN ABSORBIERBAREN IMPLANTATS**
PROCESS FOR THE PREPARATON OF A MICROSTRUCTURED ABSORBABLE IMPLANT
PROCÉDÉ POUR LA PREPARATION D'UN IMPLANT MICROSTRUCTURÉ ABSORBABLE

(30) Priorität: 16.01.2013 US 201361752992 P
(43) Veröffentlichungstag der Anmeldung: 23.07.2014
(73) Patentinhaber: Biotronik AG, 8180 Bülach (CH)
(72) Erfinder: Bayer, Ullrich, 18209 Bad Doberan (DE); Badendieck, Monika, 18147 Rostock (DE); Peters, Susanne, 18211 Admannshagen-Bargeshagen (DE); Drobek, Thomas, 18069 Rostock (DE); Anopuo, Okechukwu, 18119 Rostock (DE)
(74) Vertreter: Galander, Marcus

(56) Entgegenhaltungen:
- EP-A1- 2 668 966
- WO-A1-2005/024099
- US-A1- 2009 324 684
- US-A1- 2012 143 227

## Beschreibung

### Technisches Gebiet

Die Erfindung betrifft eine mikrostrukturierte Oberfläche aus einer bioresorbierbaren Magnesium-Legierung bevorzugt mit Zink und/oder Aluminium als Hauptlegierungselement sowie ein Verfahren zur Herstellung solcher mikrostrukturierten Oberflächen, die Verwendung solcher mikrostrukturierten Oberflächen in Implantaten, Implantate umfassend solche mikrostrukturierten Oberflächen sowie ein Verfahren zur Herstellung solcher Implantate. Hierbei kann die Erfindung als Implantat insbesondere ein Stent betreffen.

### Stand der Technik

Die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurismenstents bekannt, die zur Stützung beschädigter Gefäßwände dienen.

Stents besitzen einen rohrförmigen Grundkörper durch den der Blutfluss ungehindert weiterläuft und dessen Umfangswandung eine Stützfunktion für die Gefäßwand wahrnimmt. Der Grundkörper ist häufig als gitterartige Struktur verwirklicht, mit einer Vielzahl von einzeln und miteinander verbundenen Stegabschnitten. Der Grundkörper des Stents besteht aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzung für den Einsatz eines Werkstoffes als Implantatwerkstoff, der bei bestimmungsgemäßen Zweck mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatswerkstoffs ist weiterhin abhängig vom zeitlichen Ablauf der Reaktion des Biosystems, in das implantiert wird. So können relativ kurzfristig Reizungen und Entzündungen auftreten, die zu Gewebeveränderungen führen können. Biologische Systeme reagieren demnach in Abhängigkeit von den Eigenschaften des Implantatwerkstoffs in verschiedener Weise. Entsprechend der Reaktion des Biosystems können die Implantatswerkstoffe in bioaktive, bioinerte und degradierbare/resorbierbare Werkstoffe unterteilt werden.

Für die Zwecke der vorliegenden Erfindung sind lediglich metallische Implantatwerkstoffe für Stents von Interesse, genauer biokorrodierbare Legierungen des Elementes Magnesium mit Zink und/oder Aluminium als Hauptnebenelement. Solche Implantate aus biokorrodierbaren Legierungen können noch mit biokompatiblen Polymeren beschichtet sein.

Wie vorstehend bereits erwähnt, sollte ein Stent neben Erfüllung der erwünschten mechanischen Eigenschaften aus einem biokompatiblen Werkstoff bestehen, um Abstoßungsreaktionen zu minimieren. So werden bei etwa 70% aller perkutanen Interventionen Stents eingesetzt, in einer nicht unbeachtlichen Zahl der Fälle kommt es jedoch zu einer in-Stent Restenose aufgrund eines überschießenden neointimalen Wachstums, das durch eine starke Proliferation der arteriellen glatten Muskelzellen und eine chronische Entzündungsreaktion hervorgerufen wird. Zur Senkung der Restenoseraten werden verschiedene Lösungsansätze verfolgt.

Ein Ansatzpunkt ist die Verwendung biokorrodierbarer Metalllegierungen, denn zumeist ist eine dauerhafte Stützfunktion durch den Stent nicht notwendig; das zunächst geschädigte Gefäß kann sich regenerieren. Dementsprechend wird beispielsweise in DE 197 31 021 A1 vorgeschlagen, medizinische Implantate aus einem metallischen Werkstoff zu formen, dessen Hauptbestandteil ein Element aus der Gruppe Alkalimetalle, Erdalkalimetalle, Zink und Aluminium ist. Als besonders geeignet werden Legierungen auf Basis von Magnesium und Zink beschrieben. Nebenbestandteile der Legierungen können Mangan, Cobalt, Nickel, Chrom, Kupfer, Cadmium, Blei, Zinn, Thorium, Zirkonium, Silber, Gold, Palladium, Platin, Silizium, Calcium, Lithium, Aluminium und Zink sein. Weiterhin ist aus der DE 102 53 634 A1 der Einsatz einer biokorrodierbaren Magnesiumlegierung mit einem Anteil von Magnesium > 90%, Yttrium 3,7 - 5,5%, Seltenerdmetallen 1,5 - 4,4% und Rest < 1% bekannt, die sich insbesondere zur Herstellung einer Endoprothese, z.B. in Form eines selbstexpandierenden oder ballonexpandierbaren Stents, eignet.

Biokorrodierbare Implantate stellen somit einen aussichtsreichen Ansatz zur Minderung der Restenoserate dar. Ein Problem bei der Realisation derartiger Systeme ist das Korrosionsverhalten des Implantats. So sollte eine Fragmentbildung durch den Korrosionsprozess nach Möglichkeit bis zum Einwachsen des Implantats in die Gefäßwand unterbunden sein. Weiterhin sollte die Stützfunktion über den Zeitraum der therapeutischen Vorgabe aufrechterhalten werden.

Magnesium-Legierungen erlangen zunehmend wichtige technische Bedeutung als Werkstoff, insbesondere auch in der Medizintechnik, z.B. als Werkstoff für Implantate. Um eine kurze Übersicht über die verschiedenen Magnesiumlegierungen zu geben, soll an dieser Stelle näher auf die Einteilung und die Kennzeichnung von Legierungen und die Wirkung der wichtigsten Legierungselemente eingegangen werden. Weltweit hat sich die Kennzeichnung von Magnesiumlegierungen nach der ASTM-Norm durchgesetzt. Die Legierungen werden hierbei durch zwei Buchstaben der Hauptlegierungselemente gefolgt von deren gerundeten Gehalten in Gewichtsprozent gekennzeichnet. Die ASTM-Kurzbezeichnungen für die Legierungselemente des Magnesiums weisen diesen Legierungselementen folgende Kurzbuchstaben zu: A Aluminium; B Wismut; C Kupfer; D Cadmium; E Seltene Erden; F Eisen; H Thorium; K Zirkon; L Lithium; M Mangan; N Nickel; P Blei; Q Silber; R Chrom; S Silizium; T Zinn; W Yttrium Y Antimon; Z Zink. Nachgestellt können Buchstaben folgen, die verschiedene Entwicklungsstufen der entsprechenden Legierungen kennzeichnen (A, B, C...). Diese Buchstaben stellen meist eine Kennzeichnung des Gehaltes an Verunreinigungen dar - der Buchstabe X kennzeichnet die Legierung als Experimentallegierung. Bsp.: Bei der Legierung AZ91 D handelt es sich nach der Kennzeichnung um eine Magnesiumlegierung mit nominell 9 Gew.-% Aluminium und 1 Gew.-% Zink in der vierten Entwicklungsstufe.

Aufgrund der vielversprechenden Eigenschaften des Magnesiums als Werkstoff auch in der Medizintechnik wird schon seit langem versucht, mit Hilfe geeigneter Legierungselemente dessen Eigenschaftsprofil positiv zu beeinflussen. Die Steigerung der mechanischen Eigenschaften des Magnesiums durch Legierungszusätze beruht hierbei auf Mischkristallverfestigung, Ausscheidungsverfestigung oder Feinkornhärtung. Neben den mechanischen Eigenschaften lassen sich durch Legierungszusätze auch andere wichtige Eigenschaften wie u.a. die Korrosionsbeständigkeit, die Gießbarkeit, die Schweißeignung beeinflussen.

Im Folgenden ist die Wirkung der wichtigsten Legierungselemente des Magnesiums in alphabetischer Reihenfolge zusammengefasst.

Al: Aluminium ist das "klassische" Legierungselement für Magnesium. Durch Aluminiumzusatz werden die Zugfestigkeit und die Härte erhöht. Neben der Festigkeitssteigerung bewirkt Aluminium eine markante Verbesserung der Gießbarkeit des Magnesiums. Nachteilig ist die erhöhte Neigung zur Mikroporosität.

Ag: Silber erhöht in Verbindung mit den Metallen der Seltenen Erden in starkem Masse die Warmfestigkeit und Kriechbeständigkeit, bewirkt jedoch eine erhöhte Neigung zur Korrosion.

Be: Beryllium wird der Magnesiumschmelze in äußerst geringen Konzentrationen (< 30 ppm) zur Reduktion der Oxidationsneigung der Schmelze zugegeben.

Ca: Calcium weist einen effektiven Kornfeinungseffekt auf und erhöht die Kriechbeständigkeit. Bei der gießtechnischen Verarbeitung ist jedoch mit einer erhöhten Klebeneigung an der Form sowie einer erhöhten Heißrissanfälligkeit zu rechnen.

Mn: Der wichtigste Effekt einer Manganzugabe ist die starke Verbesserung der Korrosionsbeständigkeit (Kontrolle des Eisengehaltes durch eine Erniedrigung der Löslichkeit).

RE: Alle Seltene-Erden-Metalle (das Element Yttrium soll an dieser Stelle mit einbezogen werden) bilden mit Magnesium eutektische Zustandsdiagramme mit begrenzter Löslichkeit auf der Mg-reichen Seite, was eine Ausscheidungshärtung ermöglicht. Da sich sehr stabile Ausscheidungen bilden, erhöhen diese Elemente in starkem Maße die Warmfestigkeit und Kriechbeständigkeit der Legierungen.

Si: Die Zugabe von Silizium verschlechtert die Gießbarkeit. Da sich stabile Silizide (Mg₂Si) bilden können, kann die Kriechbeständigkeit erhöht sein.

Zn: Zink verbessert wie Aluminium die Gießbarkeit und hat eine festigkeitssteigernde Wirkung. Wie bei Aluminium steigt jedoch die Tendenz zur Mikroporosität. Höhere Gehalte (>2%) verstärken die Tendenz zur Heißrissbildung und verschlechtern die Schweißbarkeit.

Zr: Zirkon ist ein sehr effektiver Kornfeiner. Die Feinkörnigkeit führt zu einer Steigerung der Zugfestigkeit ohne ein Absinken der Dehnung. Allerdings sollte Zirkon nicht aluminium- oder siliziumhaltigen Schmelzen zugegeben werden, da durch Reaktion mit diesen Elementen die kornfeinende Wirkung verloren geht.

Während Magnesium-Legierungen als Werkstoff im Leichtbau (z.B. Fahrzeugbau, Luftfahrt, Maschinenbau, Konsumgüter) vielfach als Gusslegierungen zum Einsatz gelangen, sind in der Medizintechnik eher sogenannte Knetlegierungen gebräuchlich. Als Knetlegierungen bezeichnet man in der Metallurgie der Nichteisenmetalle Legierungen, die sich durch eine ihre Duktilität begünstigende Zusammensetzung für Aufgaben beim Walzen, Pressen, Ziehen, Schmieden von den Gusslegierungen unterscheiden, die als Flüssigmetall durch Vergießen in eine Sand- oder metallische Dauerform zu Werkstücken in Form von Gussteilen verarbeitet werden. Knetlegierungen sind ein Zwischenprodukt, auch Halbzeug genannt, bei dessen Herstellung im Vergleich mit Gusslegierungen des gleichen Typs einige Besonderheiten bestehen. In ihrer analytischen Grundzusammensetzung weichen sie von den Gusslegierungen nur wenig ab. Da die Hauptforderung an eine Knetlegierung die Eignung zu Kalt- oder Warmverformung ist, gegebenenfalls ist auch die Zerspanbarkeit von Bedeutung, kann dies sowohl die Begrenzung des Anteils einiger bei Gusslegierungen nicht störender Begleitelemente verlangen, ebenso aber die Zufügung von Elementen, die geeignet sind, die weitere Verarbeitung der Knetlegierung zu fördern.

Den guten Eigenschaften von Magnesium-Legierungen stehen auch einige negative Gesichtspunkte bei der Anwendung von Magnesium und seinen Legierungen gegenüber. Da Magnesium in der hexagonal dichtesten Kugelpackung (hdp) kristallisiert, besteht an sich eine schlechte Eignung für die Kaltverformung. Ursache hierfür ist, dass unterhalb von 225°C eine Verformung nur durch zwei unabhängige Gleitsysteme erfolgen kann und somit die Bedingung nach fünf unabhängigen Gleitsystemen (von Mises) für eine generelle Formänderung nicht erfüllt ist. Oberhalb von rund 225°C nimmt die Verformbarkeit durch Bildung neuer pyramidaler Gleitebenen nahezu sprunghaft zu - starke Verformungen sollten daher oberhalb dieser Temperatur erfolgen. Aufgrund der hexagonalen Gitterstruktur und der Neigung zur Zwillingsbildung hat sich der Werkstoff Magnesium daher bisher nur beschränkt als Knetwerkstoff etabliert. Die zusätzliche Problematik der Korrosion des Magnesiums erfordert die Entwicklung von deutlich korrosionsbeständigeren High-Purity-Legierungen mit strengen Gehaltslimiten für u.a. Eisen, Nickel und Kupfer (Fe < 0.005%, Ni < 0.001%, Cu < 0.015%). Auch aus diesem Grunde ist die verfügbare Legierungspalette des Magnesiums als Knetwerkstoff noch weiter eingeschränkt als für die Gusslegierungen. Eine bedeutende Rolle, z.B. im Leichtbau, spielen auch hier Mg-Al-Legierungen wie AZ31, AZ61 und AZ80. Zudem existieren noch Legierungen mit Zink als Hauptlegierungselement wie ZK60. Die Verarbeitung dieser Legierungen erfolgt über eine Warmumformung wie Walzen, Strangpressen und Schmieden bei Temperaturen oberhalb 350°C. Bei einer nachgeschalteten Kaltumformung können dann lediglich kleine Umformgrade toleriert werden, da es sonst zur Ausbildung von Rissen im Material kommt. Der Entwicklung von Knetlegierungen des Magnesiums gilt in jüngster Vergangenheit wieder ein verstärktes Interesse, da Magnesium-Legierungen zunehmend auch in medizintechnischen Werkstücken, z.B. als Implantate wie insbesondere Stent-Grundkörper, zum Einsatz kommen sollen.

Im Stand der Technik sind z.B. in der DE 2008 10040143 Magnesiumlegierungen beschrieben, die Yttrium und weitere Seltenerdmetalle enthalten, da sich eine derartige Legierung aufgrund ihrer physikochemischen Eigenschaften und ihrer hohen Biokompatibilität, insbesondere auch ihrer Abbauprodukte, auszeichnet. Als besonders bevorzugt werden Magnesiumlegierungen der WE-Reihe, insbesondere WE43 sowie Magnesiumlegierungen der Zusammensetzung Seltenerdmetalle 5,5-9,9 Gew.%, davon Yttrium 0,9-5,5 Gew.% und Rest ≤ 1 Gew.%, genannt, wobei der Rest Zirkonium und/- oder Silizium enthalten kann und wobei Magnesium den auf 100 Gew.% fehlenden Anteil an der Legierung einnimmt. Diese Magnesiumlegierungen bestätigten bereits experimentell und in ersten klinischen Versuchen ihre besondere Eignung, d. h. sie zeigen eine hohe Biokompatibilität, günstige Verarbeitungseigenschaften, gute mechanische Kennwerte und ein für die Einsatzzwecke adäquates Korrosionsverhalten. Unter der Sammelbezeichnung "Seltenerdmetalle" werden vorliegend Scandium (21), Yttrium (39), Lanthan (57) und die 14 an Lanthan (57) folgenden Elemente, nämlich Cer (58), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71), verstanden.

Magnesiumlegierungen für absorbierbare Stents werden oftmals mit einer oder mehreren, z.B. einer polymeren und/oder ggf. auch wirkstoffhaltigen, Beschichtungen versehen, und die Magnesiumlegierungen müssen daher Oberflächenmorphologien aufweisen, die eine ausreichende Adhäsionswirkung zur Beschichtung, z.B. zu einer polymeren Primärbeschichtung (Basecoat), zeigen. Andernfalls reißt die Beschichtung, insbesondere das Polymer, bei der Dilatation des Stents an den am meisten verformten Stellen des Stents (z.B. in den Innenseiten der Bögen) auf und das Metall wird freigelegt. Eine erhöhte Korrosion und verringerte Stützwirkung des Stents sind die Folge. Dieses Problem ist bei den bisher zur Anwendung kommenden Magnesiumlegierungen des Typs WE 43 nicht so ausgeprägt. Der Grund dafür liegt in den legierungsbedingt vorhandenen Ausscheidungen die neben den erhabenen Korngrenzen auch bei elektropolierten Oberflächen zu einer gewissen Oberflächenrauheit führen. Diese bewirkt wiederum eine befriedigende Haftung nachfolgender polymerer Deckschichten.

Andere, neuartige und vorteilhafte Magnesium-Legierungen mit Zink als wesentlichem Hauptlegierungselement, z.B Magnesium-Legierungen wie Z50 (95 Gew.-% Mg; 5 Gew.-% Zn) zeigen keine Neigung zur Ausbildung sekundärer Phasen und neigen somit legierungsbedingt weniger zu Ausscheidungen. Auch sind deren Korngrenzen nach der Elektropolitur nicht erhaben. Somit entfällt bei diesen Magnesium-Legierungen mit Zink als wesentlichem Hauptlegierungselement, aufgrund der sehr glatten Oberflächen nach der Elektropolitur, ein wesentlicher Effekt, der die Polymerhaftung begünstigt.

Im Stand der Technik sind bereits einige Versuche bekannt, die Oberflächen-Eigenschaften von Implantaten, insbesondere Stents, zu verbessern. So offenbart die US 2010/0305684 einen Mg-Stent der zur Erhöhung der Korrosionsbeständigkeit mit einer keramischen Schicht überzogen wird. Der Mg-Grundkörper erhält durch eine elektrochemische Fluorierung eine MgF₂ Zwischenschicht, die genau wie die abschließende keramische Schicht durch Vermeidung von Brüchen und Rissen die Korrosion des Mg-Grundkörpers verzögern soll. Die US 2010/0305684 enthält aber keine Angaben zur Oberflächenbeschaffenheit der durch elektrochemische Fluorierung erzeugten MgF₂ Zwischenschicht.

Weiterhin offenbart die US 2006/0198869 sehr allgemein die Herstellung biologisch abbaubarer Stents. Während der Herstellung wird geätzt und auch ein Überzug aus Polymer wird offenbart. Ebenso wird die Schaffung von Mikrostrukturen der Größe 15-250 Micrometer (µm) per Mikroätzung beschrieben. Diese Mikrostrukturen gemäß US 2006/0198869 werden jedoch nur auf dem auf die Oberfläche zusätzlich aufgebrachten Strukturelement, insbesondere durch und/oder im Polymermaterial gebildet. Eine Mikrostrukturierung der Oberfläche des Mg-Grundkörpers innerhalb der Oberfläche der Mg-Legierung und/oder der Oberfläche der Mg-Legierung selbst findet dagegen bei der US 2006/0198869 nicht statt.

Es besteht somit die Aufgabe die Oberflächen von Magnesium-Legierungen bevorzugt mit Zink und/oder Aluminium, insbesondere aber mit Zink, als wesentlichem Hauptlegierungselement so zu strukturieren, dass auch bei diesen Magnesium-Legierungen eine Rauheit erzielt wird, die eine optimale Adhäsion von Beschichtungen möglich macht, also z.B. bei einer polymeren und/oder ggf. auch wirkstoffhaltigen Primärbeschichtung eine optimale Polymeradhäsion und/oder optimale Adhäsion einer wirkstoffhaltigen Primärschicht gewährleistet.

Die erfindungsgemäße Aufgabe besteht nunmehr insbesondere darin, derartige Magnesium-Legierungen mit Zink und/oder Aluminium, insbesondere aber mit Zink, als wesentlichem Hauptlegierungselement, z.B. Magnesium-Legierungen wie Z50, oberflächlich so zu strukturieren, dass diese Oberflächen ein erhöhtes Haftungsvermögen für polymere Deckschichten aufweisen und dabei hinsichtlich des Bruchverhaltens keine Nachteile zeigen. Eine weitere Aufgabe besteht darin, die Korrosionsbeständigkeit des Grundmaterials, also der Magnesium-Legierungen bevorzugt mit Zink und/oder Aluminium, insbesondere aber mit Zink, als wesentlichem Hauptlegierungselement, an sich zu erhöhen.

### Zusammenfassung der Erfindung:

Die vorliegende Aufgabe wird durch den Gegenstand des unabhängigen Anspruchs 1 gelöst. Konkret schlägt die Erfindung zur Lösung der Aufgabe ein Verfahren zur Herstellung eines Implantats mit einer mikrostrukturierten Oberfläche aus einer bioresorbierbaren Magnesium-Legierung mit Zink und/oder Aluminium als wesentlichem Hauptlegierungselement vor. Bevorzugte Ausgestaltungen der erfindungsgemäßen Gegenstände werden in den abhängigen Ansprüchen sowie der nachfolgenden detaillierten Beschreibung dargestellt und sind, sofern für den Fachmann sinnvoll, untereinander kombinierbar.

Zusammenfassend betrifft die vorliegende Erfindung als Grundidee die Herstellung von mikrostrukturierten Oberflächen von Magnesium-Legierungen bevorzugt mit Zink und/oder Aluminium, insbesondere aber mit Zink, als Hauptlegierungselement, insbesondere bei absorbierbaren Implantaten wie z.B. Stents, wobei Mikrostrukturen der (Korn-) Größenordnung bis 10 µm (micrometer), insbesondere bis 7 µm (micrometer), und vorzugsweise bis 5 µm (micrometer), auf einem Magnesium-Legierungsgrundkörper eines absorbierbaren Implantats, z.B. eines absorbierbaren Stents, die durch Ätzen (Beizen) und elektrochemisches Mikropolieren, vorzugsweise durch kombiniertes Ätzen (Beizen) und elektrochemisches Mikropolieren, verändert werden, eine bessere Haftung für einen Polymerüberzug, höherere Beständigkeit gegen Einreißen bei mechanischer Beanspruchung (sogenannte Bruchbeständigkeit) und höhere Korrosionsbeständigkeit ermöglichen. So erhält z.B. ein absorbierbares Implantat, z.B. ein Stent, aus einer feinstkörnigen, d.h. mit einer vorstehenden (Korn-) Größenordnung im micrometer-Bereich, Magnesiumbasislegierung durch eine spezifische Oberflächenbehandlungsmethode eine mikrostrukturierte Oberfläche. Diese erfindungsgemäße mikrostrukturierte Oberfläche zeichnet sich sowohl durch ein hohes Adhäsionsvermögen für Polymerschichten als auch durch eine erhöhte Korrosionsresistenz aus. Die mikrostrukturierte Oberfläche wird aus dem Bulk-Material heraus erzeugt und zeigt bei der mechanischen Verformung des Implantates keine Delaminationserscheinungen zum Grundwerkstoff.

Die vorliegende Erfindung löst dadurch eine Reihe von Problemen und Nachteilen des Standes der Technik, wie insbesondere: die zu geringe Adhäsionswirkung zu Polymerschichten (d.h. nach außen); die zu geringe Haftung der Oberflächen zum Bulk-Material (d.h. nach innen); die negativen Auswirkungen auf die mechanischen Eigenschaften des Implantates bzw. Stents bei der Dilatation (Kerbwirkungen und daraus resultierende geringe Dilatationsdurchmesser beim ersten und zweiten Bruch); die fehlende oder nur ungenügende Korrosionsschutzwirkung. Die Erfindung löst damit insbesondere sowohl die Aufgabe der Verbesserung der Polymerhaftung auf absorbierbaren Implantaten, insbesondere auf absorbierbaren Magnesium-Stents (AMS) als auch die Aufgabe der Erhöhung der Korrosionsbeständigkeit solcher Implantate bzw. Stents. Sie verbessert dabei für die Anwendung solcher absorbierbaren Implantate, insbesondere Stents, wesentliche Eigenschaften des Grundkörpers der Magnesium-Legierung. Magnesium-Legierungen haben wegen ihrer Eigenschaft zur Biodegradation und Ihrer guten Bioverträglichkeit als Material für Implantate, und insbesondere auch Stents, großes Interesse erlangt und durch die vorliegende Erfindung können einige der bei diesen Legierungen im Stand der Technik noch bestehende Nachteile in überraschender und vorteilhafter Weise behoben werden. So ist z.B. das als Legierungszusatz bevorzugt verwendete Zink (Zn) neben Magnesium (Mg) und Calcium (Ca) ein für den Körper wichtiges Spurenelement. Auch wird z.B. Zn als Legierungszusatz - im Gegensatz zu Y und den Seltenen Erden - hinsichtlich schädlicher Wechselwirkungen bei resorbierbaren Implantaten nicht kontrovers diskutiert.

Die mikrostrukturierten Oberflächen bei Magnesium-Legierungen mit Zink und/oder Aluminium, insbesondere aber mit Zink, als Hauptlegierungselement, insbesondere bei absorbierbaren Implantaten wie z.B. Stents, lassen sich durch dem Fachmann an sich bekannte Techniken zur chemischen, metallurgischen und/oder strukturellen Untersuchung analysieren und nachweisen.

Unter einer Magnesium-Legierung wird im Rahmen der vorliegenden Erfindung immer eine Legierung mit Magnesium als Hauptkomponente verstanden. Magnesium ist entsprechend als Hauptkomponente das Element mit dem größten Gew.-% Anteil in der Legierung. Als Hauptlegierungselemente werden die Elemente bezeichnet, die nach Magnesium den größten Gew.-% Anteil der Legierung ausmachen.

### Detaillierte Beschreibung der Erfindung

Demnach betrifft die Erfindung ein Verfahren zur Herstellung einer mikrostrukturierten Oberfläche aus einer bioresorbierbaren Magnesium-Legierung mit Zink und/oder Aluminium, das sich dadurch auszeichnet, dass man eine Oberfläche einer bioresorbierbaren Magnesium-Legierung durch einen Beizprozess und einen Elektropolierprozess, vorzugsweise durch einen kombinierten Beiz- und Elektropolierprozess, behandelt, wobei
a) die zu behandelnde Oberfläche ein Gefüge aus Korn einer mittleren Korngröße von ≤ 10 µm, vorzugsweise von ≤ 7 µm, und besonders bevorzugt von ≤ 5 µm, einer bioresorbierbaren Magnesium-Legierung mit Magnesium als Hauptkomponente und mit Zink und/oder Aluminium als Hauptlegierungselement, vorzugsweise mit Zink als Hauptlegierungselement, umfasst oder daraus besteht; und wobei
b) die Magnesium-Legierung eine Zusammensetzung umfasst oder daraus besteht, die
   - Magnesium in einer Menge von 78,0 - 98,99 Gew.-%, bevorzugt 88,5 - 96,9 Gew.-%, weiter bevorzugt 91,8 - 95,8 Gew.-%, und besonders bevorzugt 94,0 - 95,4 Gew.-%; und
   - Zink und/oder Aluminium, vorzugsweise Zink, in einer Gesamtmenge von 1 - 20 Gew.-%, bevorzugt 3 - 10 Gew.-%, weiter bevorzugt 4 - 7 Gew.-%, und besonders bevorzugt 4,5 - 5,5 Gew.-%; und
   - gewünschtenfalls einen oder mehrere weitere für Magnesium-Legierungen übliche und physiologisch verträgliche Legierungszusätze insbesondere Calcium in einer Gesamtmenge von 0,01 - 2 Gew.-%, bevorzugt 0,1 - 1,5 Gew.-%, weiter bevorzugt 0,2 - 1,2 Gew.-%, und besonders bevorzugt 0,1 - 0,5 Gew.-%;
   jeweils bezogen auf die gesamte Magnesium-Legierung als 100 Gew.-%, enthält.

Bevorzugt betrifft die Erfindung das vorstehende Verfahren zur Herstellung einer mikrostrukturierten Oberfläche aus einer bioresorbierbaren Magnesium-Legierung mit Zink und/oder Aluminium, bei dem man eine Oberfläche einer bioresorbierbaren Magnesium-Legierung durch einen Beizprozess und einen Elektropolierprozess, vorzugsweise durch einen kombinierten Beiz- und Elektropolierprozess, behandelt, das sich dadurch auszeichnet, dass die Magnesium-Legierung eine Zusammensetzung umfasst oder daraus besteht, die
- Magnesium in einer Menge von 80 - 99 Gew.-%, bevorzugt 90 - 97 Gew.-%, weiter bevorzugt 93 - 96 Gew.-%, und besonders bevorzugt 94,5 - 95,5 Gew.-%; und
- Zink und/oder Aluminium, vorzugsweise Zink, in einer Menge von 1 - 20 Gew.-%, bevorzugt 3 - 10 Gew.-%, weiter bevorzugt 4 - 7 Gew.-%, und besonders bevorzugt 4,5 - 5,5 Gew.-%;
- und gewünschtenfalls einen oder mehrere weitere für Magnesium-Legierungen übliche und physiologisch verträgliche Legierungszusätze, insbesondere Calcium in einer Gesamtmenge von 0,01 - 2 Gew.-%, bevorzugt 0,1 - 1,5 Gew.-%, weiter bevorzugt 0,2 - 1,2 Gew.-%, und besonders bevorzugt 0,1 - 0,5 Gew.-%;
jeweils bezogen auf die gesamte Magnesium-Legierung als 100 Gew.-%, enthält.

Erfindungsgemäß wird also die Oberfläche eines Magnesium-Legierungsgrundkörpers, d.h.. eines absorbierbaren Implantats, insbesondere z.B. eines absorbierbaren Stents, durch Ätzen (ggf. auch als "Beizen" oder "Beizprozess" bezeichnet) und elektrochemisches Mikropolieren verändert. Sowohl Ätzen (Beizen) als auch das Elektropolieren sind dem Fachmann an sich geläufige Methoden, die auch in der Medizintechnik bekannt sind und vielseitige Anwendung finden.

Unter Ätzen bzw. einem Beizprozess im Sinne der vorliegenden Erfindung versteht man ein Verfahren, dass in der Regel in Säuren oder in säurehaltigen Gemischen stattfindet und eine intensive Reinigung und Aktivierung von Metalloberflächen bewirkt, meist verbunden mit einem leichten Werkstoffabtrag. Das Ergebnis sind metallisch reine Oberflächen, frei von Oxiden und Kontamination durch Fremdmetall. Ätzen bzw. Beizen greift bevorzugt die Bereiche eines Metallgefüges, an die im Vergleich zum benachbarten Bereich eine gegenüber der jeweiligen Ätz- bzw. Beizlösung geringere Korrosionsbeständigkeit aufweisen. Dies können z.B. Körner sein, die gegenüber dem jeweiligen Nachbarkorn eine leicht veränderte Legierungszusammensetzung aufweisen. Bevorzugt werden hierbei Körner chemisch angegriffen, in welchen das oder die die Korrosionsbeständigkeit der Legierung erhöhenden Legierungselemente in einer geringeren Konzentration vorliegen als in den übrigen Körnern. Ätzen bzw. Beizen kann sowohl chemisch als auch elektrolytisch (anodisch) erfolgen. Ätz- bzw. Beizmittel enthalten in der Regel Mineralsäuren und ein Oxidationsmittel sowie Zusätze zur Verbesserung des Ätz- bzw. Beizergebnisses und zur Reduzierung von Schadstoffen.

Unter einem Elektropolierprozess (Elektropolieren) versteht man elektrochemische Verfahren zum Erzeugen und Optimieren einer Vielzahl technisch-funktioneller und/oder dekorativer Eigenschaften sowie zur Erzielung von Gratfreiheit und/oder Partikelfreiheit von Oberflächen durch Behandlung in einem Elektrolyten unter Anlegen einer elektrischen Spannung. Elektropolierverfahren sind industriell erprobt und dem Fachmann als zuverlässig und wirtschaftlich für vielfältige Anwendungsbereiche bekannt, insbesondere überall dort, wo erhöhte Anforderungen an Funktion und Aussehen metallischer Oberflächen bestehen. Elektropolieren wirkt hierbei im Mikrobereich, ohne Formen und Makrostrukturen zu verändern, wobei es ohne mechanische oder thermische Belastung des behandelten Werkstoffs durch anodische Auflösung eine dünne Werkstoffschicht von der Werkstoffoberfläche abträgt. Alle darin enthaltenen Verunreinigungen, Partikel, Mikrorisse, Gefügestörungen und lokalen Spannungen werden damit beseitigt. Der durch Feldlinienverdichtungseffekte hervorgerufene erhöhte Abtrag an Ecken und Kanten bewirkt deren zuverlässige Entgratung und Glättung. Elektropolierte Oberflächen sind grat- und partikelfrei, metallisch rein, glänzend, im Mikrobereich glatt und geschlossen und weisen die optimalen Eigenschaften des Grundwerkstoffs auf.

"Kombiniert" im Sinne der vorliegenden Erfindung für den Ätz- und Elektropolierprozess bedeutet nicht, dass beide Prozesse gleichzeitig erfolgen müssen. Letzteres ist jedoch bevorzugt. Das Ätzverfahren ("Ätzen", auch als "Beizen" oder "Beizprozess" bezeichnet) und das elektrochemische Mikropolieren können einerseits als separate Verfahren, z.B. wenn ein bereits (vor-)geätzter Magnesium-Legierungsgrundkörper erst später oder ein bereits elektropolierter Magnesium-Legierungsgrundkörper nochmals durch Elektropolieren bearbeitet oder nachbearbeitet werden soll, und andererseits vorzugsweise auch durch gleichzeitiges kombiniertes Ätzen und elektrochemisches Mikropolieren durchgeführt werden. Erfolgt der Ätz- und Elektropolierprozess bevorzugt gleichzeitig kombiniert, ist der Übergang vom Ätzen zum Elektropolieren durch Anlegen der Spannung für den Elektropolierprozess gekennzeichnet.

Erfindungsgemäß ist die (Korn-)Größenordnung der Mikrostrukturen im micrometer-Bereich bis 10 µm (micrometer), insbesondere bis 7 µm (micrometer), und vorzugsweise bis 5 µm (micrometer), von Bedeutung, insbesondere angegeben als statistisches Mittel. So weisen die Korngrößen besonders bevorzugter erfindungsgemäßer Magnesiumlegierungen ein statistisches Mittel von 1 bis 5 µm auf, d.h. dass 95 % aller gemessenen Körner in diesem Größenbereich vorliegen. Die mittlere Korngröße wird dabei nach dem Verfahren der DIN EN ISO 643:2003 bestimmt. Dabei kommen zur Bestimmung der Korngröße genormte Bildreihentafeln aus ASTM E 112 zur Anwendung (Kontaktstelle für Vergleichsbilder ist ASTM, 100 Barr Harbor Drive, Philadelphia, PA 19428-3914, USA; Referenz-Nr. ADJ 12-501120-10).

Unter dem Begriff "Mikrostruktur" wird im Rahmen der vorliegenden Erfindung eine dreidimensionale Oberflächenlandschaft bzw. Oberflächenstruktur im micrometer-Bereich verstanden, die durch ein Gefüge aus Korn einer (Korn-)Größenordnung bis 10 µm, insbesondere bis 7 µm, und vorzugsweise bis 5 µm, das nach Herauslösen von einzelnem Korn aus der Oberfläche (z.B. durch Beizen und Elektropolieren) gebildet wird. Diese dreidimensionale Oberflächenlandschaft bzw. Oberflächenstruktur, d.h. das an der Oberfläche vorliegende dreidimensionale Gefüge aus Korn der vorstehenden Größenordnung, weist an der Oberfläche Vertiefungen (Krater) mit einer in die Oberfläche hineinragenden maximalen Tiefe von etwa 3 µm (Kratertiefe) und einem maximalen seitlichen Durchmesser bis 10 µm (Kraterbreite) auf. Diese dreidimensionale Oberflächenlandschaft bzw. Oberflächenstruktur ist somit eine Kraterstruktur mit 3 µm maximaler Kratertiefe und 10 µm maximaler Kraterbreite. Die Erfindung betrifft daher auch ein Verfahren zur Herstellung einer mikrostrukturierten Oberfläche, das sich dadurch auszeichnet, dass die erzeugte mikrostrukturierte Oberfläche eine Mikrostruktur aus einem Gefüge aus Korn mit erhabenen Korngrenzen und benachbarten Vertiefungen aufweist; vorzugsweise eine Mikrostruktur aus einem Gefüge aus Korn mit erhabenen Korngrenzen und benachbarten Vertiefungen, die als kraterförmige Mikrostrukturen ausgebildet sind. Bevorzugte erfindungsgemäße Verfahren zur Herstellung einer mikrostrukturierten Oberfläche zeichnen sich auch dadurch aus, dass eine mikrostrukturierte Oberfläche mit einer Mikrostruktur aus einem Gefüge aus Korn erzeugt wird, die einer mittleren Korngröße von 1 bis 8 µm, vorzugsweise von 2 bis 7 µm, weiter bevorzugt von 3 bis 6 µm, und besonders bevorzugt von 3 bis 5 µm, entspricht.

Im Sinne der vorliegenden Erfindung bedeutet der Begriff "degradierbarer Magnesium-Stent", dass der Grundkörper des Magnesium-Stents in physiologischer Umgebung, insbesondere im Gefäßsystem eines menschlichen oder tierischen Körpers degradiert, d.h. so abgebaut wird, dass der Stent seine Integrität verliert. Erfindungsgemäß degradiert der Magnesium-Stent erst dann, wenn die Funktion des Stents nicht mehr physiologisch sinnvoll, bzw. notwendig ist, d.h. wenn das traumatisierte Gewebe des Gefäßes verheilt ist und der Stent nicht länger im Gefäßlumen verbleiben muss.

Gemäß der vorliegenden Erfindung umfasst oder besteht der degradierbare Magnesium-Stent bzw. Magnesium-Stentgrundkörper aus einer biokorrodierbaren Magnesium-Legierung. Unter Magnesium-Legierung wird vorliegend ein metallisches Gefüge verstanden, dessen Hauptkomponente in der Legierung Magnesium ist. Hauptkomponente ist die Legierungskomponente, deren Gewichtsanteil an der Legierung am höchsten ist. Neben Magnesium als Hauptlegierungskomponente enthalten die Legierungen gemäß vorliegender Erfindung Zink und/oder Aluminium als Nebenlegierungskomponente. Besonders bevorzugt sind hierbei Magnesium-Legierungen mit Zink als hauptsächliche Nebenlegierungskomponente.

Die Magnesium-Legierung umfassend Magnesium als Hauptkomponente sowie Zink und/oder Aluminium, vorzugsweise Zink, als Hauptlegierungselement ist im Sinne der Erfindung so in ihrer Zusammensetzung zu wählen, dass sie biokorrodierbar ist. "Biokorrodierbar" und "bioabsorbierbar" werden in diesem Text analog verwendet. Erfindungsgemäß besteht der metallische Grundkörper des Implantats bzw. des Stents aus einer biokorrodierbaren Magnesium-Legierung mit Zink und/oder Aluminium, vorzugsweise Zink, als weiterer Legierungskomponente. Als biokorrodierbar im Sinne der Erfindung werden Legierungen bezeichnet, bei denen in physiologischer Umgebung ein Abbau/Umbau stattfindet, so dass der aus der Legierung bestehende Teil eines Implantates, insbesondere eines Stents, ganz oder zumindest überwiegend nicht mehr vorhanden ist. Die Legierung ist demnach so in ihrer Zusammensetzung zu wählen, dass sie biokorrodierbar ist. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCl 0,4 g/l, MgSO₄ 0,1 g/l, NaHCO₃ 2,2 g/l, Na₂HPO₄ 0,126 g/l, NaH₂PO₄ 0,026 g/l). Eine Probe der zu untersuchenden Legierung wird dazu in einem verschlossenen Probenbehälter mit einer definierten Menge des Prüfmediums bei 37 ° C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Erfindungsgemäß besteht der Grundkörper aus einer biokorrodierbaren Magnesium-Legierung. Bevorzugt enthält die biokorrodierbare Magnesiumlegierung neben Magnesium und Zink und/oder Aluminium für Magnesiumlegierungen übliche Legierungszusätze. Als weitere Legierungselemente kommen ein oder mehrere Metalle aus der Gruppe bestehend aus Mangan (Mn), Silber (Ag), Cer (Ce), Silizium (Si), Zirkonium (Zr), Lithium (Li) und Calcium (Ca) in Frage. Bei Mg-Legierungen mit Zink als Hauptlegierungselement kann auch Aluminium (Al) bzw. bei Mg-Legierungen mit Aluminium als Hauptlegierungselement kann auch Zink (Zn) zur vorstehenden Gruppe weiterer Legierungselemente gerechnet werden. Besonders bevorzugt sind als diese weiteren Legierungszusätze Calcium (Ca) und/oder Zirkon (Zr), da sich derartige Legierungen aufgrund ihrer physikochemischen Eigenschaften und hohen Biokompatibilität, insbesondere auch ihrer Abbauprodukte, auszeichnen.

Seltenerdmetalle sind als weiterer Legierungszusatz eher von untergeordneter Bedeutung für die vorliegende Erfindung und Legierungen mit höheren Gehalten an Seltenerdmetallen sind ggf. sogar ungeeignet. Unter der Sammelbezeichnung "Seltenerdmetalle" werden vorliegend Scandium (21), Yttrium (39), Lanthan (57) und die 14 auf Lanthan (57) folgenden Elemente, nämlich Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71) verstanden. Solche Seltenerdmetalle bzw. die anderen weiteren Legierungselemente können, wie nachfolgend erläutert, im Rahmen der Erfindung in bestimmten Mengen in den Magnesium-Legierungen mit Zink und/oder Aluminium, vorzugsweise Zink, als wesentliches Hauptlegierungselement vorliegen, wobei sich die Angaben auf Gew.% an der Legierung beziehen und Magnesium und Zink und/oder Aluminium den auf 100 Gew.% verbleibenden Restanteil an der Legierung einnehmen. Der Fachmann weiß hierbei, dass diese Mengenangaben wegen herstellungsbedingter Verunreinigungen geringfügig variieren können und er ist in der Lage, diese ggf. geringfügigen Abweichungen richtig einzuordnen; z.B. bei der Beurteilung, ob eine Zusammensetzung in einen angegebenen Bereich fällt.

Im Rahmen der Erfindung sollte bevorzugt die Gesamtmenge an Seltenerdmetallen in den Legierungen, jeweils bezogen auf die gesamte Legierung als 100 Gew.-%, nicht mehr als 3 Gew.-%, vorzugsweise nicht mehr als 2 Gew.-%, besonders bevorzugt nicht mehr als 1 Gew.-%, betragen. Die Menge an Seltenerdmetallen kann daher im Bereich von 1 bis 3 Gew.-%, vorzugsweise von 1 bis 2 Gew.-%, und besonders bevorzugt im Bereich von 0 bis 1 Gew.-%, liegen, wobei Magnesium und Zink und/oder Aluminium zusammen den auf 100 Gew.% fehlenden Anteil an der Legierung ausmachen.

Im Rahmen der Erfindung sollte die Menge an Legierungselementen, jeweils bezogen auf die gesamte Legierung als 100 Gew.-%, ausgewählt aus der Gruppe Zink (Zn), Aluminium (Al), Mangan (Mn), Silber (Ag), Cer (Ce), Silizium (Si), Zirkonium (Zr), Calcium (Ca) und Lithium (Li), vorzugsweise aus der Gruppe Zink (Zn), Aluminium (Al), Calcium (Ca) und Zirkonium (Zr) in den Legierungen nicht mehr als 11 Gew.-%, vorzugsweise nicht mehr als 8 Gew.-%, besonders bevorzugt nicht mehr als 7 Gew.-%, betragen. Die Gesamtmenge an diesen weiteren Legierungselementen kann daher im Bereich von 0 bis 11. Gew.-%, vorzugsweise von 0 bis 8 Gew.-%, und besonders bevorzugt im Bereich von 0 bis 7 Gew.-%, liegen, wobei Magnesium den auf 100 Gew.% fehlenden Anteil an der Legierung ausmacht.

In einer beispielhaften Ausgestaltung der Erfindung weist die Magnesium-Legierung eine Zusammensetzung aus 94,75 Gew.-% Magnesium, 5 Gew.-% Zink und 0,25 Gew.-% Calcium auf. In einer weiteren beispielhaften Ausgestaltung der Erfindung weist die Magnesium-Legierung eine Zusammensetzung aus 95 Gew.-% Magnesium und 5 Gew.-% Zink auf.

Bevorzugt sind weiterhin Magnesium-Legierungen die Zink in einer Menge bis zu 11 Gew.%, insbesondere Zink in einer Menge von 2-10 Gew.%, enthalten. Besonders bevorzugt sind weiterhin Magnesiumlegierungen, die neben Magnesium und Zink in der vorstehenden Menge in der Zusammensetzung zusätzlich weitere übliche Legierungszusätze enthalten, die in ihrer Summe ≤ 2 Gew.-% der Gesamtlegierung ausmachen. Beispiele hierfür sind Zusammensetzungen, die Calcium in einer Menge von 0,05-1 Gew.% und/oder Yttrium in einer Menge von 0,5-1 Gew.% und/oder Mangan in einer Menge von 0-0,5 Gew.% und/oder Silber in einer Menge von 0-1 Gew.% und/oder Cer in einer Menge von 0-1 Gew.% und/oder Zirkonium in einer Menge von 0-1 Gew.% und/oder Silizium in einer Menge von 0-0,4 Gew.% enthalten und/oder Lithium einer Menge von 0-1 Gew.%, wobei sich die Angaben auf Gew.% an der gesamten Legierung beziehen und Magnesium sowie herstellungsbedingte Verunreinigungen den auf 100 Gew.% verbleibenden Restanteil an der Legierung einnehmen.

Stents weisen grundsätzlich eine Tragstruktur auf, die geeignet ist, die Wand eines Gefäßes in geeigneter Weise abzustützen, um so das Gefäß zu weiten bzw. ein Aneurisma zu überbrücken. Stents werden dazu in einem komprimierten Zustand in das Gefäß eingeführt und dann an dem zu behandelnden Ort aufgeweitet und gegen die Gefäßwand gedrückt. Dieses Aufweiten kann beispielsweise mit Hilfe eines Ballonkatheters erfolgen. Alternativ sind auch selbstexpandierende Stents bekannt. Diese sind beispielsweise aus einem superelastischen Metall, wie Nitinol, aufgebaut. Solche Stents können mit einer erfindungsgemäß mikrostrukturierten Oberfläche versehen sein.

Stents werden derzeit in zwei Grundtypen eingeteilt, die dauerhaften Stents und die degradierbaren Stents. Dauerhafte Stents sind so ausgestaltet, dass sie im Gefäß für einen unbestimmten Zeitraum verbleiben können. Degradierbare Stents hingegen werden über einen vorbestimmten Zeitraum hinweg in einem Gefäß abgebaut. Vorzugsweise werden degradierbare Stents erst abgebaut, wenn das traumatisierte Gewebe des Gefäßes verheilt ist und somit der Stent nicht weiter im Gefäßlumen verbleiben muss. Als degradierbare Stentmaterialien sind beispielsweise degradierbare Metalllegierungen, Polymere oder Verbundwerkstoffe, die eine ausreichende strukturelle Tragfähigkeit aufweisen, um das Gefäß lumen über einen vorbestimmten Zeitraum stützen zu können, bekannt.

Im Sinne der vorliegenden Erfindung können als erfindungsgemäß degradierbarer Magnesium-Stent alle üblichen Stentgeometrien verwendet werden. Insbesondere bevorzugt sind Stentgeometrien, die in US 6,896,695 , US 2006/241742 , US 5,968,083 (Tenax) EP 1 430 854 (Helix-Design), US 6,197,047 und EP 0 884 985 beschrieben werden.

Der Magnesium- Stent kann vorzugsweise mit einem Coating (Beschichtung), ggf. auch als zusätzliches Topcoat, versehen sein, umfassend oder bestehend aus einem oder mehreren biodegradierbaren Polymeren und/ oder deren Blends wie z.B. PLLA, PLGA und PCL.

Die Anmeldung betrifft auch die Bereitstellung einer mikrostrukturierten Oberfläche aus einer bioresorbierbaren Magnesium-Legierung bevorzugt mit Zink und/oder Aluminium, vorzugsweise mit Zink, als Hauptlegierungselement wobei sich die mikrostrukturierte Oberfläche dadurch auszeichnet, dass
a) die Oberfläche, ein Gefüge aus Korn einer mittleren Korngröße von ≤ 10 µm, vorzugsweise von ≤ 7 µm, und besonders bevorzugt von ≤ 5 µm, einer bioresorbierbaren Magnesium-Legierung mit Magnesium als Hauptkomponente und mit Zink und/oder Aluminium als Hauptlegierungselement, vorzugsweise mit Zink als Hauptlegierungselement, umfasst oder daraus besteht; und wobei
b) die Magnesium-Legierung eine Zusammensetzung umfasst oder daraus besteht, die
   - Magnesium in einer Menge von 78,0 - 98,99 Gew.-%, bevorzugt 88,5 - 96,9 Gew.-%, weiter bevorzugt 91,8 - 95,8 Gew.-%, und besonders bevorzugt 94,0 - 95,4 Gew.-%; und
   - bevorzugt Zink und/oder Aluminium, vorzugsweise Zink, in einer Gesamtmenge von 1 - 20 Gew.-%, bevorzugt 3 - 10 Gew.-%, weiter bevorzugt 4 - 7 Gew.-%, und besonders bevorzugt 4,5 - 5,5 Gew.-%; und
   - gewünschtenfalls einen oder mehrere weitere für Magnesium-Legierungen übliche und physiologisch verträgliche Legierungszusätze in einer Gesamtmenge von 0,01 - 2 Gew.-%, bevorzugt 0,1 - 1,5 Gew.-%, weiter bevorzugt 0,2 - 1,2 Gew.-%, und besonders bevorzugt 0,1 - 0,5 Gew.-%;
   jeweils bezogen auf die gesamte Magnesium-Legierung als 100 Gew.-%, enthält wobei bevorzugt die mikrostrukturierte Oberfläche eine Mikrostruktur aus erhabenen Korngrenzen und benachbarten Vertiefungen umfasst oder daraus besteht; vorzugsweise eine Mikrostruktur aus erhabenen Korngrenzen und benachbarten Vertiefungen, die als kraterförmige Mikrostrukturen ausgebildet sind.

Insbesondere umfasst ist hierbei eine mikrostrukturierte Oberfläche, bei der die Magnesium-Legierung eine Zusammensetzung umfasst oder daraus besteht, die
- Magnesium in einer Menge von 80 - 99 Gew.-%, bevorzugt 90 - 97 Gew.-%, weiter bevorzugt 93 - 96 Gew.-%, und besonders bevorzugt 94,5 - 95,5 Gew.-%;
- Zink und/oder Aluminium, vorzugsweise Zink, in einer Menge von 1 - 20 Gew.-%, bevorzugt 3 - 10 Gew.-%, weiter bevorzugt 4 - 7 Gew.-%, und besonders bevorzugt 4,5 - 5,5 Gew.-%; und
- gewünschtenfalls einen oder mehrere weitere für Magnesium-Legierungen übliche und physiologisch verträgliche Legierungszusätze in einer Gesamtmenge von 0,01 - 2 Gew.-%, bevorzugt 0,1 - 1,5 Gew.-%, weiter bevorzugt 0,2 - 1,2 Gew.-%, und besonders bevorzugt 0,1 - 0,5 Gew.-%;
jeweils bezogen auf die gesamte Magnesium-Legierung als 100 Gew.-%, enthält.

Weitere bevorzugte mikrostrukturierte Oberflächen zeichnen sich dadurch aus, dass die mikrostrukturierte Oberfläche eine Mikrostruktur aus einem Gefüge aus Korn umfasst oder daraus besteht, die einer Korngröße von 1 bis 8 µm, vorzugsweise von 2 bis 7 µm, weiter bevorzugt von 3 bis 6 µm, und besonders bevorzugt von 3 bis 5 µm, entspricht.

Die Anmeldung betrifft weiterhin die Verwendung einer mikrostrukturierten Oberfläche und die Bereitstellung eines Implantates sowie auch Implantate umfassend eine vorstehend beschriebene mikrostrukturierte Oberfläche aus einer bioresorbierbaren Magnesium-Legierung bevorzugt mit Zink und/oder Aluminium, vorzugsweise mit Zink, als Hauptlegierungselement. Solche Implantate umfassend eine vorstehend beschriebene mikrostrukturierte Oberfläche aus der bioresorbierbaren Magnesium-Legierung bevorzugt mit Zink und/oder Aluminium, vorzugsweise mit Zink, als Hauptlegierungselement können mit einer Beschichtung aus einem oder mehreren bioverträglichen Polymeren, vorzugsweise aus einem oder mehreren bioresorbierbaren Polymeren, versehen sein. Insbesondere ist ein solches erfindungsgemäßes Implantat ein Stent.

Weitere Details zur erfindungsgemäßen Lösung der Aufgabe bzw. der vorliegenden Erfindung sollen nachfolgend noch detaillierter erläutert werden.

Der Erfindung liegt die kombinierte Erkenntnis zugrunde, dass sowohl eine kleinere durchschnittliche Korngröße in einem Gefüge einer Magnesium-Legierung als auch Körner mit einem unterdurchschnittlichen Gehalt des Nebenlegierungsbestandteils Zink und/oder Aluminium im feinkörnigen Gefüge der Magnesium-Legierung zu einer erhöhten Korrosionsrate in diesen Bereichen führt. Die sogenannte Kornfeinung entsteht hierbei dadurch, dass z.B. durch Wärmebehandlung bei der Herstellung, z.B. bei Extrudieren, eines Werkstoffs aus Magnesium-Legierung ein kleineres, feineres Korn im Gefüge der Magnesium-Legierung gebildet wird. Gleichzeitig können hierbei einerseits Bereiche mit Korn und/oder Feinkorn im Gefüge der Magnesium-Legierung gebildet werden, worin die Körner einen unterdurchschnittlichen Gehalt des Nebenlegierungsbestandteils Zink und/oder Aluminium aufweisen, und andererseits können Bereiche mit Korn und/oder Feinkorn im Gefüge der Magnesium-Legierung gebildet werden, worin die Körner einen etwas höheren Zink- und/oder Aluminium-Gehalt als den mittleren (durchschnittlichen) Zink- bzw. Aluminium-Legierungsgehalt aufweisen. In einem Werkstoff aus einer Magnesium-Legierung mit Zink und/oder Aluminium als Nebenlegierungsbestandteil, können so Bereiche mit unterschiedlicher durchschnittlicher Korngröße und/oder unterschiedlichem lokalen (Korn-bezogenen) Zink- und/oder Aluminium-Gehalt und damit mit unterschiedlichen Korrosionseigenschaften gebildet werden.

Erfindungsgemäß ist nun vorgesehen, diese Unterschiede im Korrosionsverhalten von Bereichen eines Werkstoffs auf Basis von Magnesium- Legierungen mit Zink und/oder Aluminium als Hauptlegierungselement in Abhängigkeit seiner Korngröße und/oder des lokalen (Korn-bezogenen) Zink- und/oder Aluminium-Gehaltes zu nutzen, um einerseits durch gezielte Korrosion insgesamt eine mikrostrukturierte Oberfläche aus einer biokorrodierbaren, Zink und/oder Aluminium als Hauptlegierungselement enthaltenden Magnesium-Legierung mit erhöhter Korrosionsfestigkeit zu erzielen und hierdurch insbesondere auch das Korrosionsverhalten, den Degradationsvorgang und die Degradationszeit eines Implantates, vorzugsweise eines Stents, umfassend eine solche mikrostrukturierte Oberfläche, vorteilhaft bzw. vorab kalkulierbarer zu beeinflussen; andererseits ist erfindungsgemäß auch vorgesehen das Korrosionsverhalten zu nutzen, um die Adhäsionswirkung für aufzubringende Polymerbeschichtungen zu verbessern.

Der Zeitraum für die Degradation beginnt unmittelbar nach der Implantation und endet zu einem vorgebbaren Zeitpunkt, der den therapeutischen Vorgaben und Anforderungen an die Sicherheit entspricht. Vorzugsweise erstreckt sich dieser Zeitraum über 2 bis 6 Wochen unmittelbar nach der Implantation. So ist in der Regel z.B. ein Stent innerhalb dieses Zeitraums in die Gefäßwand eingewachsen und diese ist soweit regeneriert, dass eine weitere Stützfunktion nicht mehr notwendig ist.

Für die Erfindung besonders geeignete Korngrößen und Kornzusammensetzungen weisen dabei Magnesium-Legierungen mit Zink und/oder Aluminium als Hauptlegierungselement auf, die Knetlegierungen sind. Die Erfindung betrifft daher auch ein Verfahren zur Herstellung einer mikrostrukturierten Oberfläche bei dem die eingesetzte Magnesium-Legierung eine Knetlegierung ist und/oder eine heterogene und/oder keine kristallographische Vorzugsausrichtung aufweist. Als Knetlegierungen bezeichnet man in der Metallurgie der Nichteisenmetalle Legierungen, die sich durch eine ihre Duktilität begünstigende Zusammensetzung für Aufgaben beim Walzen, Pressen, Ziehen, Schmieden von Gusslegierungen unterscheiden. Als solche duktile Zusammensetzungen sind auch die Knetlegierungen im Sinne der vorliegenden Erfindung zu verstehen.

Der Begriff Korrosion bezieht sich vorliegend auf die Reaktion des metallischen Werkstoffes mit seiner Umgebung, wobei eine messbare Veränderung des Werkstoffs bewirkt wird, die - bei Einsatz des Werkstoffs in einem Bauteil - zu einer Beeinträchtigung der Funktion des Bauteils führt. Ein Korrosionssystem besteht aus dem korrodierenden metallischen Werkstoff sowie einem (flüssigen) Korrosionsmedium, das in seiner Zusammensetzung die Verhältnisse in physiologischer Umgebung nachstellt oder ein physiologisches Medium, insbesondere Blut ist. Werkstoffseitig wird die Korrosion durch Faktoren beeinflusst, wie die Zusammensetzung und Vorbehandlung der Legierung, mikro- und submikroskopische Inhomogenitäten, Randzoneneigenschaften, Temperatur- und Spannungszustand und insbesondere die Zusammensetzung einer die Oberfläche bedeckenden Schicht. Auf Seiten des Mediums wird der Korrosionsprozess durch Leitfähigkeit, Temperatur, Temperaturgradienten, Azidität, Volumen-OberflächenVerhältnis, Konzentrationsunterschied sowie Strömungsgeschwindigkeit beeinflusst.

In einem weiteren Aspekt bezieht sich die Erfindung auf ein Verfahren zur Herstellung eines erfindungsgemäßen Stents, umfassend die Schritte
a) Bereitstellen eines Stents oder eines Stent-Vorläuferproduktes als extrudierte Hülse mit AD = 2,00 mm und WD von 190 µm;
b) Behandlung des Stents oder eines Stent-Vorläuferproduktes durch Laserschneiden der extrudierten Hülse zu einer lasergeschnittenen Stentstruktur;
c) Reibahlen der lasergeschnittenen Stentstruktur zum Entfernen von der an den Laserschnittkanten anhaftenden Schlacke;
d) erfindungsgemäßes Ätzen (Beizen) und Elektroplieren.

In diesem Verfahren kommen Stents oder Stent-Vorläuferprodukte mit einer Struktur aus einer biokorrodierbaren Magnesium-Legierung zum Einsatz.

Absorbierbare Stents aus Magnesium-Legierungen wie beispielsweise Z50 (95 Gew.-% Mg; 5 Gew.-% Zn) und andere werden im Rahmen der Erfindung bevorzugt mittels eines Extrusionsprozesses hergestellt. Durch die spezifische Verfahrensführung der Extrusion kommt es z.B. zur Ausbildung eines sehr feinkörnigen Gefüges im micrometer-Bereich wie oben angegeben von ≤ 10 µm, ≤ 7 µm oder ≤ 5 µm, und insbesondere mit einer mittleren Korngröße von 1 bis 5 µm, vorzugsweise von 2 bis 4 µm, in der Magnesium-Legierung. Das so gefertigte hülsenförmige Halbzeug wird anschließend mittels Laserschneiden zu einer stentförmigen Struktur geschnitten. Danach erfolgt erfindungsgemäß ein, vorzugsweise kombinierter, Beiz- und Elektropolierprozess der zu einer mikrostrukturierten Oberfläche der Magnesium-Legierung führt. Der Beizangriff erfolgt derart, dass benachbarte Körner unterschiedlich stark abgetragen werden. Dieser Effekt basiert auf unterschiedlicher kristallographischer Orientierung der Körner im Gefüge der Magnesium-Legierung sowie auf deren Unterschiede im Gehalt an gelöstem Zn und/oder Al.

Die Erfindung betrifft daher auch ein Verfahren zur Herstellung einer mikrostrukturierten Oberfläche bei dem die zu behandelnde Oberfläche eine Magnesium-Legierung aus einem Gefüge aus Korn umfasst oder daraus besteht, worin verschiedenes Korn mit zueinander unterschiedlichem Zinkgehalt und/oder Aluminiumgehalt vorliegt. In diesem Verfahren zur Herstellung einer mikrostrukturierten Oberfläche zeichnet sich die zu behandelnde Oberfläche der Magnesium-Legierung insbesondere durch ein Gefüge aus Korn einer mittleren Korngröße von 1 bis 10 µm, vorzugsweise von 1 bis 7 µm, und besonders bevorzugt von 1 bis 5 µm, und weiter bevorzugt von 2 bis 4 µm, aus.

### Durch die vorstehend beschriebene selektive Ätzung werden nunmehr zwei grundlegende Effekte erzielt:

Erstens, weist die entstehende Oberfläche Mikrostrukturbreiten auf, die z.B. mit denen der mittleren Korngröße von 1-5 µm, insbesondere von 2-4 µm, korrespondieren. Das bedeutet, dass kristallographisch günstig orientierte Körner der Magnesium-Legierung mit einem etwas höheren Zn- bzw. Al-Anteil als der mittlere Zn- bzw. Al-Legierungsgehalt nicht (oder weniger) angegriffen werden, die benachbarten Körner jedoch zum Teil oder ganzheitlich weggebeizt werden. Somit entstehen, zumindest in der Oberfläche der Magnesium-Legierung bzw. des Implantates oder Stents, im Querschnitt bevorzugt kraterförmige Strukturen, die einerseits hohe Kohäsionskräfte zum darunter (innen) befindlichen Bulk-Material und andererseits eine extrem hohe Adhäsionswirkung zu nachfolgend (außen) auf der Oberfläche der Magnesium-Legierung bzw. dem Implantat oder Stent aufgebrachten Polymerschichten aufweisen.

Zweitens, wird durch die chemische Entfernung von weniger resistenten Oberflächenbereichen mittels Beizen und Elektropolieren die integrale Korrosionsfestigkeit der Oberfläche der Magnesium-Legierung bzw. des Implantates oder des Stents erhöht. Dies bewirkt eine längere Standzeit der Oberfläche der Magnesium-Legierung bzw. des Implantates oder des Stents unter physiologischen Bedingungen, und zwar unabhängig davon, ob eine polymere Beschichtung (Coating) zur Anwendung kommt oder nicht.

Überraschenderweise hat sich gezeigt, dass die erfindungsgemäße Kombination von einer Legierungszusammensetzung mit der oben und in den Beispielen angegebenen Korngröße des Bulk-Materials, insbesondere bei einer darin aufgrund Extrusionsprozess (z.B. Fließpressen) und anschließender Rekristallisationsglühung fehlender kristallographischer Vorzugsausrichtung der Körner in der Legierungszusammensetzung, mit dem zur Anwendung kommenden Oberflächenbehandlungsverfahren, sicher und reproduzierbar zu den erfindungsgemäßen mikrostrukturierten Oberflächen mit den genannten Vorteilen führen.

Ein wichtiges Verfahren zur Oberflächenstrukturierung ist das Polieren. Als Resultat sollte es bei vernachlässigbaren Substrateffekten atomistisch flache Oberflächen liefern. Aufgrund unterschiedlicher Reaktivität der Körner erfolgt allerdings ein stark kornabhängiger Metallabtrag. Manche Körner sind nach dem Polieren höher als andere, wobei die resultierenden Höhendifferenzen zwischen den Kristalliten abhängig sind von: Polierprozess, Polierdauer und Kombination der Orientierung benachbarter Körner sowie von der Zusammensetzung der einzelnen Körner.

Die folgende Modelbetrachtung beschreibt die Oberflächenelemente, die durch einen unterschiedlichen Polierprozess auftreten können, z.B. im Vergleich von chemisch- und elektrochemisch poliertem Metall. Beobachtet werden auf den Körnern kleine Hügel und Löcher, an den Korngrenzen Wälle, Gräben oder Stufen. Das Auftreten der verschiedenen Oberflächenelemente ist orientierungsabhängig, wobei die Orientierung der Kristallite in der Regel willkürlich ist. Die Orientierungen der Körner können mittels EBSD (Electron Back Scattered Diffraction) bestimmt und die Rauhigkeit sowie die Topographie mittels Rasterkraftmikroskopie (AFM). Das Untersuchungsergebnis kann üblicherweise mittels EBSD-Mapping des untersuchten Probenbereichs von elektrochemisch poliertem Metall (für entsprechende Polierbedingungen) dargestellt werden.

Die Elektropolitur, auch Elektropolieren genannt, zählt zu den abtragenden Fertigungsverfahren. Genauer wird es den elektrochemischen Abtragverfahren mit Fremdstromquelle zugeordnet. Dabei wird in einem speziell auf das Material abgestimmten Elektrolyten Metall anodisch abgetragen, das heißt, das metallische Werkstück bildet die Anode in einer elektrochemischen Zelle. Das elektrochemische Abtragen von Teilen der Oberfläche soll hier kurz skizziert werden. Der Abtrag findet zumeist mit Gleichstrom statt, doch auch der Einsatz gepulster Ströme ist möglich. Das Werkstück wird anodisch geschaltet. Industriell werden Stromdichten angelegt, die einen Abtrag im transpassiven Bereich der Stromdichte-Spannungs-Kurve ermöglichen. Dies hat zur Folge, dass nicht nur Metall abgetragen wird, sondern auch Sauerstoff an der Anode, dem Werkstück, entsteht.

Übliche Elektrolyte sind häufig Mischungen aus Mineralsäuren und Wasser sowie in einigen Fällen Alkoholen. Gemische aus Phosphorsäure und Schwefelsäure werden für das Elektropolieren von Edelstählen und Stählen sowie Aluminiumlegierungen eingesetzt. Messing und Kupfer lassen sich in Mischungen aus Phosphorsäure und Alkoholen bearbeiten. Mischungen aus 55 Gew.-% Phosphorsäure und 35 Gew.-% Schwefelsäure und eignen sich auch für das Elektropolieren von Aluminium. Für Kupfer und Messing eignen sich wässrige Elektrolyte aus 50 Gew.-% Phosphoräure und 30 Gew.-% Alkohol, beispielsweise 2-Propanol. Es wird ein speziell ausgewählter Elektrolyt eingesetzt, um die Oberflächen von Magnesium-Legierungen mit Zink und/oder Aluminium, vorzugsweise Zink, als wesentliches Hauptlegierungselement einer Elektropolitur zu unterziehen. Insbesondere betrifft die Erfindung daher ein Verfahren zur Herstellung einer mikrostrukturierten Oberfläche, bei dem der Beizprozess und/oder Elektropolierprozess, vorzugsweise der kombinierte Beiz- und Elektropolierprozess, unter Verwendung eines Phosphorsäure-haltigen Elektrolyten ausgeführt wird. So ist ein Elektrolyt aus 20 Vol.-% deionisiertes Wasser, 30 Vol.-% Phosphorsäure (85 Gew.-%), und 50 Vol.-% Ethanol (99 Gew.-%) sehr gut geeignet. Im erfindungsgemäßen Verfahren zur Herstellung einer mikrostrukturierten Oberfläche wird daher insbesondere ein Elektrolyt mit folgender Zusammensetzung, bezogen auf 100 Vol.-% der Elektrolytzusammensetzung, verwendet: 15 bis 25 Vol.-% deionisiertes Wasser, 25 bis 35 Vol.-% Phosphorsäure (85 ± 5 gew.-%ig) und 45 bis 55 Vol.-% Ethanol (50 ± 5 gew.-%ig); vorzugsweise etwa 20 Vol.% deionisiertes Wasser, etwa 30 Vol.-% Phosphorsäure (85 ± 5 gew.-%ig) und etwa 50 Vol.% Ethanol (50 ± 5 gew.-%ig).

Des Weiteren können den Elektrolytbädern vielfach oberflächenaktive Substanzen zugemischt werden. Die Elektrolyte sind in den meisten Fällen Gefahrstoffe. Dementsprechend ist sachkundiger Umgang mit den Stoffen erforderlich, um Gesundheits- und Umweltschäden zu vermeiden.

Effekt der vorliegenden Erfindung ist es, jedoch im Gegensatz zum eigentlichen Ziel einer Elektropolitur, die Rauheit der Oberfläche von bestimmten Magnesium-Legierungen, die Zink und/oder Aluminium als Hauptlegierungselement enthalten, zu erhöhen, um die z.B. Adhäsion von Polymeren zu verbessern, ohne jedoch die Korrosionsbeständigkeit der Legierung zu vermindern.

Überraschenderweise wurde nun erfindungsgemäß gefunden, dass eine Kombination aus Beizen und elektrochemischem Polieren die Ziele der Erfindung bei den genannten Magnesium-Legierungen vorteilhaft erfüllt. Ein chemisches, elektrolytisches und/oder elektrochemisches Beizen ist erfindungsgemäß somit zwingend erforderlich, um mit der Elektropolitur kombiniert zu werden oder diese zu ergänzen.

Unter Beizen versteht man im technischen Bereich die Behandlung von festen Körpern zur Veränderung der Oberfläche mit einer Beize. Dieses Beizen dient u. a. zum Schutz der Oberfläche gegen Oxidation (bei Metall). Das Beizen geschieht im Stand der Technik in der Hauptsache durch ein Anätzen mittels aggressiver Chemikalien, meist Säuren oder Laugen. Der Vorgang wird unter anderem in der Galvanotechnik eingesetzt, um aufgetragene Metallschichten zu entfernen oder um eine oxidfreie Oberfläche zu bekommen. Oft wird der Vorgang durch elektrischen Strom unterstützt. In der Galvanotechnik kommen diverse stromlose und stromunterstützte Beizverfahren zum Einsatz. Der Grund ist meist die Aktivierung des Grundmetalls für die weitere Beschichtung. Die Aktivierung ist für jedes Grundmetall bzw. Legierung unterschiedlich. Oft können schon kleine Unterschiede der Legierungsbestandteile unterschiedliche Beizverfahren erfordern, insbesondere bei so sensiblen Werkstoffen, die in der Medizintechnik eingesetzt werden sollen.

Überraschenderweise gelingt es durch die erfindungsgemäße Kombination von Beizen und Elektropolieren die Rauheit von glatten und sogar sehr glatten Oberflächen unter Ausbildung einer mikrostrukturierten Oberfläche vorteilhaft, insbesondere für Implantat- und Stent-Anwendungen, zu erhöhen, wodurch die Adhäsion von Polymeren verbessert wird und gleichzeitig sogar die Korrosionsbeständigkeit der Legierung insgesamt, d.h. auch ohne Polymerbeschichtung zunimmt. Die verbesserte Polymerhaftung äußert sich z.B. darin, dass nachträglich mittels Sprühen oder Dipcoating aufgebrachte absorbierbare Polymere wie PLGA, PLLA, PCL und deren Blends auch bei maximaler mechanischer Belastung des Stents ihre Adhäsion zum Bulk-Material der Legierung nicht einbüßen. Das bedeutet, dass bei einem vorgegebenen Design Stents bis zu Durchmessern von 5,2 mm dilatiert werden können und die Polymerschichten zeigen keine Risse, keine Löcher und delaminieren nicht. Dies führt beispielsweise im Vergleich zu nur elektropolierten Referenzstents zu einer um mindestens 4 Wochen verlängerten Degradationszeit. Gegebenenfalls können auch Polysaccharide zum Einsatz kommen. Hierbei ist ein bevorzugtes Beispiel Polylactid-co-Glycolid (PLGA) eine organische Substanz auf Milchsäurebasis, die vom menschlichen Körper leicht abgebaut werden kann. PLGA als bevorzugtes Beispiel wird als chirurgisches Nähmaterial eingesetzt. PLLA (polylactic acid oder polylactide) als ebenfalls bevorzugtes Beispiel ist ein thermoplastisches aliphatisches Polyesterderivat aus erneuerbaren Ressourcen wie z.B. Maisstärke (in den USA), Wurzeln bzw. Abfälle oder Stärke aus Tapioka (meist in Asien), oder Zuckerrohr (restliche Welt). PLLA ist unter bestimmten Bedingungen biodegradierbar, wie z.B. in Gegenwart von Sauerstoff. PCL (Polycaprolactone) ist ein biodegradierbarer Polyester mit einem niedrigen Schmelzpunkt von etwa 60 °C und einer Glassübergangstemperatur von etwa -60°C.

Die Erfindung weist somit eine Reihe von technischen Vorteilen gegenüber den schon vorbekannten Magnesium-Legierungen und deren Oberflächen auf, wie insbesondere: die Degradationszeit wird verlängert; der Degradationsvorgang wird weniger von Fehlstellen beeinflusst; die Degradationszeit wird dadurch besser kalkulierbar (Streuung wird verringert); eine Wirkstoffelution wird nicht durch frühzeitige Bulk-Korrosion beeinträchtigt. Insgesamt wird durch diese unerwarteten und vorteilhaften Eigenschaften der Gebrauchswert medizinisch-technischer Produkte, wie z.B. Implantate oder Stents deutlich erhöht.

Nachfolgend wird die Erfindung anhand von Ausführungsbeispielen näher erläutert.

### 1. Beispiel:

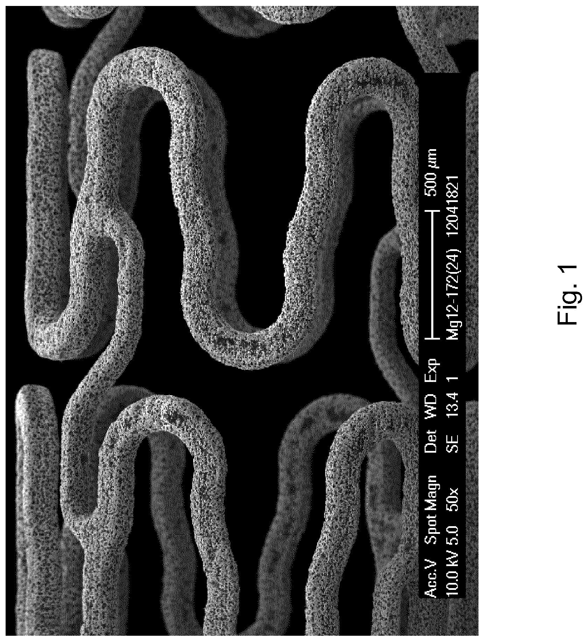

Hülsen aus der Magnesiumlegierung mit 94,75 Gew.-% Mg, 5,0 Gew.-% Zn und 0,25 Gew.-% Ca wurden zwischen 200 °C und 300 °C mittels Vorwärtshohlfließpressen und Umformgeschwindigkeiten zwischen 0,5/s und 3/s extrudiert. Die Extrusion erfolgte in einer Fliesspressvorrichtung bestehend aus dem den Innenteil (Stempel) und dem Außenteil (Matrize). Die Hülsengeometrie war gekennzeichnet durch einen Außendurchmesser zwischen 1,6 mm und 2,4 mm sowie einer Wandstärke zwischen 140 µm und 210 µm. Das Halbzeug wurde mittels Laserschneiden und nachfolgenden Prozessen wie Reibahlen zu einer Stentgeometrie weiter verarbeitet. Als Reibahlen bezeichnet man hierbei eine spanabhebendes Fertigungsverfahren das zur Feinbearbeitung von Bohrungen (vor allem in Metallteilen) durch Reiben verwendet wird. Verbessert werden sowohl die Oberflächengüte als auch die Form- und Maßgenauigkeit. Zur Erzielung der erfindungsgemäßen Oberfläche erfolgte eine Kombination aus Ätzen (Beizen) und Elektropolieren in einem phosphorsäurehaltigem Elektrolyt über eine Gesamtzeit von ca. 2 min. Die reibgeahlten Stents wurden auf Titandrähte aufgefädelt und in die Beizlösung getaucht (siehe Beispiel 4). Danach lag die geforderte Stentgeometrie hinsichtlich Stegbreite (ca. 100 µm) und Wanddicke (ca. 120 µm) vor. Um nunmehr den gewünschten Mikrostruktureffekt zu erzielen, wurde für 10 bis 30 s im selben Elektrolyt ein finaler Beizprozess durchgeführt. Dieser führte zur Mikrostrukturierung der Oberfläche, die auch ein Herauslösen der Körner mit geringer Korrosionsfestigkeit aus der Oberfläche einschließt (siehe Figur 1).

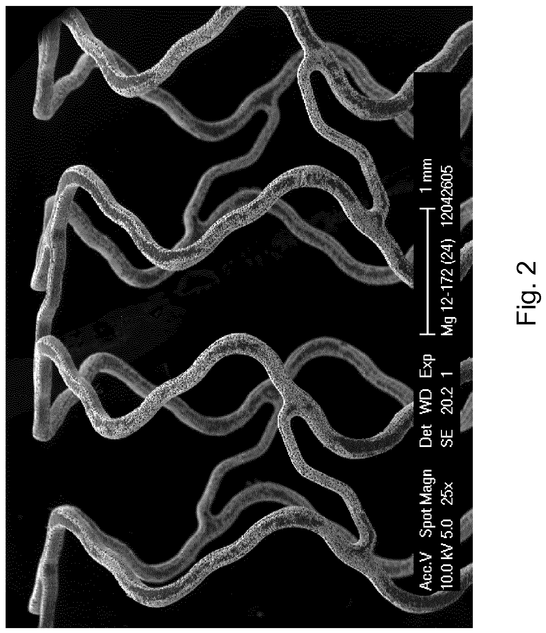

- Figur 1:: Mikrostrukturierte Stentoberfläche nach Ausführungsbeispiel 1 (Rasterelektronenmikroskopie). Diese Stentoberfläche des gleichen Stents nach dem Dilatieren von 2,00 mm auf 4,00 mm wird im Figur 2 gezeigt.
- Figur 2:: Nach der Dilatation des Stents von Bild 1 auf 4,00 mm Durchmesser (Rasterelektronenmikroskopie). Es zeigt sich, dass die mikrostrukturierte Oberfläche erhalten bleibt und keine mechanischen Beschädigungen vorliegen.

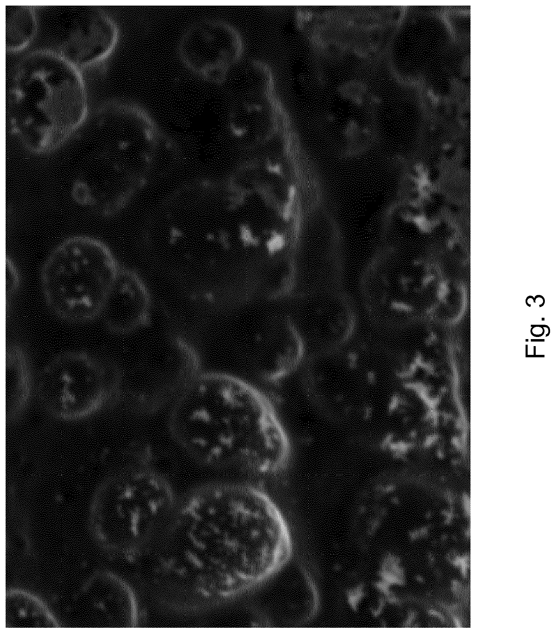

- Figur 3:: Detail der mikrostrukturierten Oberfläche mit herausgelösten Körnern (Rasterelektronenmikroskopie).

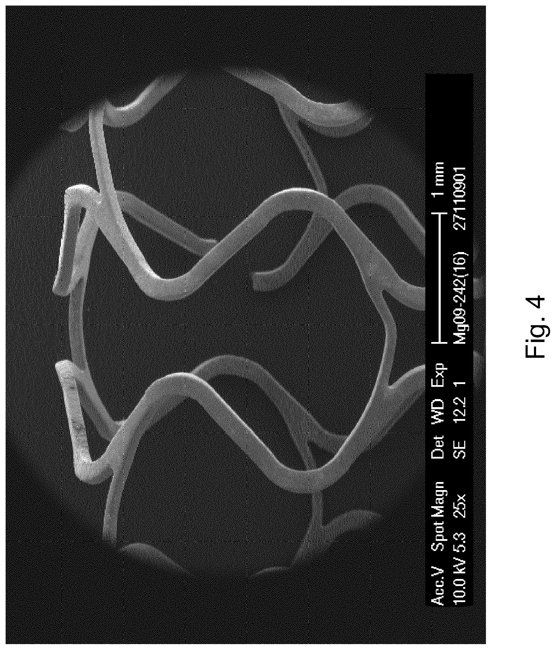

- Figur 4:: Vergleichsbild einer Oberfläche ohne Mikrostrukturierung (Rasterelektronenmikroskopie).

Die verbesserte Polymerhaftung äußerte sich darin, dass nachträglich mittels Sprühen oder Dipcoating aufgebrachte absorbierbare Polymere wie PLGA, PLLA, PCL und deren Blends auch bei maximaler mechanischer Belastung des Stents ihre Adhäsion zum Bulk nicht einbüßten. Das bedeutet, dass bei einem vorgegebenen Design Stents bis zu Durchmessern von 5,2 mm dilatiert werden konnten und die Polymerschichten keine Risse und keine Löcher zeigten und nicht delaminierten. Dies führte zu einer - im Vergleich zu nur elektropolierten Referenzstents - um mindestens 4 Wochen verlängerten Degradationszeit.

### 2. Beispiel:

Durchführung wie im Beispiel 1 angegeben für eine Legierung ohne Calcium, d.h. für eine Legierung mit 95,0 Gew.-% Mg, und 5,0 Gew.-% Zn.

### 3. Beispiel:

Durchführung wie im Beispiel 1, jedoch mit einer dem Extrusionsprozess nachgeschalteten Wärmebehandlung 250°C über 5 min an Luft.

Hülsen aus der Magnesiumlegierung mit 94,75 Gew.-% Mg, 5,0 Gew.-% Zn und 0,25 Gew.-% Ca wurden zwischen 200 °C und 300 °C mittels Vorwärtshohlfließpressen und Umformgeschwindigkeiten zwischen 0,5/s und 3/s wie in Beispiel 1 extrudiert. Die Extrusion erfolgte in einer Fließpressvorrichtung bestehend aus dem den Innenteil (Stempel) und dem Außenteil (Matrize). Die Hülsengeometrie war gekennzeichnet durch einen Außendurchmesser zwischen 1,6 mm und 2,4 mm sowie einer Wandstärke zwischen 140 µm und 210 µm. Das Halbzeug wurde mittels Laserschneiden und nachfolgenden Prozessen wie Reibahlen zu einer Stentgeometrie weiterverarbeitet. Zur Erzielung der erfindungsgemäßen Oberfläche erfolgte eine Kombination aus Beizen und Elektropolieren in einem phosphorsäurehaltigem Elektrolyt durch Tauchbeizen (siehe Beispiel 4) über eine Gesamtzeit von ca. 2 min.

Die auf dem Titandraht befindlichen Stents wurden zunächst für ca. 45 s chemisch gebeizt (d.h. ohne Strom). Danach erfolgte, ohne dass die Stents das Beizbad verlassen, das Anlegen einer Spannung von 6 V über einen Zeitraum von 1 min. Die Stents waren dabei anodisch geschaltet.

Ein Stent mit einem spezifischen Stentdesign hat bei einer Länge von ca. 20 mm eine Gesamtoberfläche von ca. 150 mm². Bei einer Spannung von 6 V liegt eine Stromdichte von ca. 0,05 bis 0,15 A/cm² an. Nach einer Zeit von wiederum 45 s wurde der Stromfluss unterbrochen. Der Stent hatte nun nahezu die gewünschten Endabmessungen und zeigte eine relativ glatte Oberfläche.

Es lag die geforderte Stentgeometrie hinsichtlich Stegbreite (ca. 100 µm) und Wanddicke (ca. 120 µm) vor. Um nunmehr den gewünschten Mikrostruktureffekt zu erzielen, wurde für 10 bis 30 s im selben Elektrolyt ein finaler Beizprozess durchgeführt. Dieser führte zur Mikrostrukturierung der Oberfläche die auch ein Herauslösen der Körner mit geringer Korrosionsfestigkeit aus der Oberfläche einschließt (siehe z.B. Figur 1).

Erfindungsgemäß wurden eine Reihe von technischen Vorteilen der Lösung gegenüber den schon vorbekannten Magnesium-Legierungen und deren Oberflächen beobachtet, wie insbesondere: die Degradationszeit wird verlängert; der Degradationsvorgang wird weniger von Fehlstellen beeinflusst; die Degradationszeit wird dadurch besser kalkulierbar (Streuung wird verringert); eine Wirkstoffelution wird nicht durch frühzeitige Bulk-Korrosion beeinträchtigt. Insgesamt wurde durch diese unerwarteten und vorteilhaften Eigenschaften Gebrauchswert medizinisch-technischer Produkte, wie z.B. Implantate oder Stents deutlich erhöht.

### 4. Beispiel:

### Beschreibung der Elektropolitur und des Beizens von Magnesium-Stents

Benötigt wird eine Elektropoliereinrichtung, bestehend aus: Elektrolytbecken und zwei Spülbecken.

Benötigt wird auch ein Elektrolyt aus: 20 Vol.-% deionisiertes Wasser, 30 Vol.-% Phosphorsäure (85 Gew.-%), und 50 Vol.-% Ethanol (99 Gew.-%). Beispielhaft kann der Elektrolyt folgende Zusammensetzung ausgedrückt in Liter aufweisen: 20 Liter deionisiertes Wasser, 30 Liter Phosphorsäure (85 Gew.-%), und 50 Liter Ethanol (99 Gew.-%).

Der Elektrolyt wird mindestens 2 Tage vor dem Einsatz in einem Kanister angemischt und ruht bis zur Verwendung.

Benötigt werden weiterhin Reinigungslösungen, insbesondere: Dimethylsulfoxid (99,5 Gew.-%) für Reinigungsbecken 1; Isopropanol (99,8 Gew.-%) für Reinigungsbecken 2.

Schließlich werden noch benötigt: ein Poliergestell, bestehend aus 6 Titanstreben mit 25 Titandrähten in 4 Reihen jeweils 5 Drähte übereinander angeordnet; Stromquellen; eine Excentervorrichtung; eine halbautomatische Reinigungsanlage.

Zur Durchführung der Elektropolitur von Magnesium-Stents werden die Stents auf die Titandrähte gefädelt, die Drähte werden durch einen zusätzlichen Draht auf der Strebe fixiert. Der Elektrolyt wird vor jedem Polierdurchlauf mit einem Rührer vermischt. Die Excentervorrichtung wird in das Elektrolytbecken eingehängt und die Stromquelle, die den Excenter antreibt, eingeschaltet. Die Polierzeit richtet sich nach dem gewünschten Abtrag und liegt zwischen 60 und 300 Sekunden.

In der ersten Hälfte der Gesamtpolierzeit findet die chemische Politur statt und in der zweiten Hälfte die Elektropolitur, indem durch zwei Krokodilklemmen das Poliergestell mit einer Spannungsquelle verbunden wird. Die Spannungsquelle wird mit 6 Volt betrieben, die Stromstärke variiert durch die Anzahl der Stents auf dem Poliergestell.

Nach Ablauf der Polierzeit wird das Gestell mit den Stents von der Spannungsquelle getrennt und aus der Elektrolytlösung herausgenommen und in ein Reinigungsbecken 1, gefüllt mit Dimethylsulfoxid (99,5 %) eingesetzt. Der Reinigungsprozess dauert 2 Minuten. Dann wird das Gestell zur Endreinigung der polierten Stents für 2 Minuten in ein zweites Reinigungsbecken mit Isopropanol gehängt.

Danach wird das Gestell mit den Stents aus der Elektropolituranlage entnommen, die fixierten Drähte wieder gelöst und es wird noch vorhandenes Isopropanol vom Stent entfernt. Der Stent trocknet dann noch ca. 3 bis 5 Minuten bei Raumtemperatur auf dem Draht. Der getrocknete Stent wird vorsichtig mit einem Stentabnehmer vom Draht entfernt und verschiedenen Prüfungen wie z.B. Vermessung und Sichtprüfung zugeführt.

## Patentansprüche

1. Verfahren zur Herstellung eines Implantats aus einer bioresorbierbaren Magnesium-Legierung mit Zink und/oder Aluminium als Hauptlegierungselement umfassend eine mikrostrukturierte Oberfläche, **dadurch gekennzeichnet, dass** man die Oberfläche durch einen Beizprozess und einen Elektropolierprozess, vorzugsweise durch einen kombinierten Beiz- und Elektropolierprozess, behandelt, wobei die Oberfläche
a) ein Gefüge aus Korn einer mittleren Korngröße von ≤ 10 µm, vorzugsweise von ≤ 7 µm, und besonders bevorzugt von ≤ 5 µm, einer bioresorbierbaren Magnesium-Legierung mit Magnesium als Hauptkomponente und mit Zink und/oder Aluminium als Hauptlegierungselement, vorzugsweise mit Zink als Hauptlegierungselement, umfasst oder daraus besteht; und wobei
b) die Magnesium-Legierung eine Zusammensetzung umfasst oder daraus besteht, mit
- Magnesium in einer Menge von 80 - 99 Gew.-%, bevorzugt 90 - 97 Gew.-%, weiter bevorzugt 93 - 96 Gew.-%, und besonders bevorzugt 94,5 - 95,5 Gew.-%; und mit
- Zink und/oder Aluminium, vorzugsweise Zink, in einer Gesamtmenge von 1 - 20 Gew.-%, bevorzugt 3 - 10 Gew.-%, weiter bevorzugt 4 - 7 Gew.-%, und besonders bevorzugt 4,5 - 5,5 Gew.-%; und
- gewünschtenfalls mit einem oder mehreren weiteren für Magnesium-Legierungen übliche Legierungszusätze in einer Gesamtmenge von 0,01 - 2 Gew.-%, bevorzugt 0,1 - 1,5 Gew.-%, weiter bevorzugt 0,2 - 1,2 Gew.-%;
wobei die vorstehenden Gew.-%-Angaben auf die gesamte Magnesium-Legierung als 100 Gew.-% bezogen sind.

2. Verfahren nach Anspruch 1, **dadurch gekennzeichnet, dass** die Magnesium-Legierung eine heterogene und/oder keine kristallographische Vorzugsausrichtung aufweist.

3. Verfahren nach Anspruch 1 oder 2, **dadurch gekennzeichnet, dass** die Magnesium-Legierung eine Kristallographie besitzt, die nicht oder nur unwesentlich zur Ausbildung sekundärer Phasen und/oder nicht oder nur unwesentlich zu Ausscheidungen neigt.

4. Verfahren nach einem der vorangehenden Ansprüche, **dadurch gekennzeichnet, dass** die zu behandelnde Oberfläche der Magnesium-Legierung ein Gefüge aus Korn umfasst oder daraus besteht, worin Korn mit zueinander unterschiedlichem Zinkgehalt und/oder Aluminiumgehalt vorliegt.

5. Verfahren nach einem der vorangehenden Ansprüche, **dadurch gekennzeichnet, dass** die zu behandelnde Oberfläche der Magnesium-Legierung ein Gefüge aus Korn einer mittleren Korngröße von 1 bis 5 µm, vorzugsweise von 2 bis 4 µm, aufweist und/oder die Legierung eine Knetlegierung ist.

6. Verfahren nach einem der vorangehenden Ansprüche, **dadurch gekennzeichnet, dass** die mikrostrukturierte Oberfläche eine Mikrostruktur aus einem Gefüge aus Korn mit erhabenen Korngrenzen und benachbarten Vertiefungen aufweist, vorzugsweise eine Mikrostruktur aus einem Gefüge aus Korn mit erhabenen Korngrenzen und benachbarten Vertiefungen, die als kraterförmige Mikrostrukturen ausgebildet sind.

7. Verfahren nach einem der vorangehenden Ansprüche, **dadurch gekennzeichnet, dass** eine mikrostrukturierte Oberfläche mit einer Mikrostruktur aus einem Gefüge aus Korn erzeugt wird, die einer mittleren Korngröße von 1 bis 8 µm, vorzugsweise von 2 bis 7 µm, besonders bevorzugt von 3 bis 6 µm, entspricht.

8. Verfahren nach einem der vorangehenden Ansprüche, **dadurch gekennzeichnet, dass** der kombinierte Beiz- und Elektropolierprozess unter Verwendung eines Phosphorsäure-haltigen Elektrolyten ausgeführt wird.

9. Verfahren nach einem der vorangehenden Ansprüche, **dadurch gekennzeichnet, dass** ein Elektrolyt verwendet wird, mit einer Zusammensetzung aus: 15 bis 25 Vol.-%, vorzugsweise etwa 20 Vol.%, deionisiertes Wasser; 25 bis 35 Vol.-%, vorzugsweise etwa 30 Vol.-% Phosphorsäure (85 %); und 45 bis 55 Vol.-%, vorzugsweise etwa 50 Vol.% Ethanol (50 %); bezogen auf 100 Vol.-% der Elektrolytzusammensetzung.

10. Verfahren nach einem der vorangehenden Ansprüche, , **dadurch gekennzeichnet, dass** die mikrostrukturierte Oberfläche mit wenigstens einem bioverträglichen, vorzugsweise mit wenigstens einem bioresorbierbaren, Polymer beschichtet ist.

11. Verfahren nach einem der vorangehenden Ansprüche, **dadurch gekennzeichnet, dass** das Implantat ein Stent ist.

## Claims

1. A method for producing an implant from a bioresorbable magnesium alloy containing zinc and/or aluminium as primary alloying element, said implant comprising a microstructured surface, **characterised in that** the surface is treated by a pickling process and an electropolishing process, preferably by a combined pickling and electropolishing process, wherein the surface comprises or consists of
a) a grain structure having a mean grain size of ≤10 µm, preferably ≤7 µm, and particularly preferably ≤5 µm, a bioresorbable magnesium alloy containing magnesium as the primary component and containing zinc and/or aluminium as the primary alloying element(s), preferably containing zinc as the primary alloying element; and wherein
b) the magnesium alloy comprises or consists of a composition containing
- magnesium in an amount of 80-99 % by weight, preferably 90-97 % by weight, more preferably 93-96 % by weight, and particularly preferably 94.5-95.5 % by weight; and containing
- zinc and/or aluminium, preferably zinc, in a total quantity of 1-20 % by weight, preferably 3- 10 % by weight, more preferably 4-7 % by weight, and particularly preferably 4.5-5.5% by weight; and
- optionally containing one or more further alloying additions that are customary for magnesium alloys, in a total quantity of 0.01-2 % by weight, preferably 0.1-1.5 % by weight, more preferably 0.2 to 1.2 % by weight,
wherein the above values in % by weight are based on the total magnesium alloy as 100 % by weight.

2. The method according to claim 1, **characterised in that** the magnesium alloy has a heterogeneous and/or no preferred crystallographic orientation.

3. The method according to claim 1 or 2, **characterised in that** the crystallography of the magnesium alloy does not tend, or tends only insignificantly, toward the formation of secondary phases and/or does not tend, or tends only insignificantly, toward precipitations.

4. The method according to any one of the preceding claims, **characterised in that** the surface of the magnesium alloy to be treated comprises or consists of a grain structure in which grains having differing zinc contents and/or aluminium contents are present.

5. The method according to any one of the preceding claims, **characterised in that** the surface of the magnesium alloy to be treated comprises a grain structure having a mean grain size of 1 to 5 µm, preferably of 2 to 4 µm, and/or the alloy is a wrought alloy.

6. The method according to any one of the preceding claims, **characterised in that** the microstructured surface has a microstructure made of a grain structure having raised grain boundaries and neighbouring depressions, preferably a microstructure made of a grain structure having raised grain boundaries and neighbouring depressions formed as crater-shaped microstructures.

7. The method according to any one of the preceding claims, **characterised in that** a microstructured surface is generated, which has a microstructure that is formed of a grain structure having a mean grain size of 1 to 8 µm, preferably of 2 to 7 µm, and particularly preferably of 3 to 6 µm.

8. The method according to any one of the preceding claims, **characterised in that** the combined pickling and electropolishing process is carried out using a phosphoric acid-containing electrolyte.

9. The method according to any one of the preceding claims, **characterised in that** an electrolyte is used having a composition selected from: 15 to 25 % by volume, preferably approximately 20% by volume, of deionised water; 25 to 35 % by volume, preferably approximately 30 % by volume, of phosphoric acid (85%); and 45 to 55 % by volume, preferably approximately 50 % by volume, of ethanol (50%); based on 100 % by volume of the electrolyte composition.

10. The method according to any one of the preceding claims, **characterised in that** the microstructured surface is coated with at least one biocompatible polymer, preferably with at least one bioresorbable polymer.

11. The method according to any one of the preceding claims, **characterised in that** the implant is a stent.

## Revendications

1. Procédé de fabrication d'un implant à base d'un alliage de magnésium bio-résorbable avec du zinc et/ou de l'aluminium en tant qu'élément d'alliage principal, comprenant une surface micro-structurée, **caractérisé en ce qu'**on traite la surface par un procédé de décapage et un procédé de polissage électrolytique, de préférence par un procédé combiné de décapage et de polissage électrolytique, où la surface
a) comprend ou est constituée d'une structure en grains avec une taille de grains moyenne ≤ 10 µm, de préférence, ≤ 7 µm, et de manière particulièrement préférée ≤ 5 µm, d'un alliage de magnésium bio-résorbable avec du magnésium en tant que composant principal et avec du zinc et/ou de l'aluminium en tant qu'élément d'alliage principal, de préférence, avec du zinc en tant qu'élément d'alliage principal ; et où
b) l'alliage de magnésium comprend ou est constitué d'une composition avec
- du magnésium en une quantité de 80 à 99 % en poids, de préférence de 90 à 97 % en poids, plus préférentiellement de 93 à 96 % en poids et de manière particulièrement préférée de 94,5 à 95,5 % en poids ; et avec
- du zinc et/ou de l'aluminium, de préférence, du zinc, en une quantité totale de 1 à 20 % en poids, de préférence de 3 à 10 % en poids, plus préférentiellement de 4 à 7 % en poids et de manière particulièrement préférée de 4,5 à 5,5 % en poids ; et
- éventuellement, avec un ou plusieurs autres additifs d'alliage usuels pour les alliages de magnésium en une quantité totale de 0,01 à 2 % en poids, de préférence de 0,1 à 1,5 % en poids, plus préférentiellement de 0,2 à 1,2 % en poids ;
où les indications en % en poids présentées se rapportent à l'alliage de magnésium total égal à 100 % en poids.

2. Procédé selon la revendication 1, **caractérisé en ce que** l'alliage de magnésium présente une orientation préférentielle hétérogène et/ou aucune orientation préférentielle cristallographique.

3. Procédé selon la revendication 1 ou 2, **caractérisé en ce que** l'alliage de magnésium possède une cristallographie qui a tendance à ne pas former de phases secondaires ou uniquement de manière minime et/ou à ne pas avoir tendance à se précipiter ou uniquement de manière minime.

4. Procédé selon l'une des revendications précédentes, **caractérisé en ce que** la surface traitée de l'alliage de magnésium comprend une structure à base de grains, ou en est constituée, où les grains sont présents avec une teneur en zinc et/ou une teneur en aluminium variables l'une par rapport à l'autre.

5. Procédé selon l'une des revendications précédentes, **caractérisé en ce que** la surface de l'alliage de magnésium à traiter présente une structure à base de grains d'une taille moyenne de grain de 1 à 5 µm, de préférence, de 2 à 4 µm, et/ou l'alliage est un alliage forgeable.

6. Procédé selon l'une des revendications précédentes, **caractérisé en ce que** la surface micro-structurée présente une microstructure à base d'une structure en grains avec des joints de grains de qualité supérieure et des cavités voisines, de préférence, une microstructure à base d'une structure en grains avec des joints de grains de qualité supérieure et des cavités voisines qui sont conçues sous forme de microstructures en forme de cratères.

7. Procédé selon l'une des revendications précédentes, **caractérisé en ce qu'**une surface micro-structurée est créée avec une microstructure à base d'une structure en grains qui correspond à une taille moyenne de grains de 1 à 8 µm, de préférence de 2 à 7 µm, de manière particulièrement préférée de 3 à 6 µm.

8. Procédé selon l'une des revendications précédentes, **caractérisé en ce que** le procédé combiné de décapage et de polissage électrolytique est effectué moyennant l'emploi d'un électrolyte contenant de l'acide phosphorique.

9. Procédé selon l'une des revendications précédentes, **caractérisé en ce qu'**un électrolyte est employé avec une composition à base de : 15 à 25 % en volume, de préférence environ 20 % en volume d'eau dé-ionisée ; 25 à 35 % en volume, de préférence environ 30 % en volume d'acide phosphorique (85 %) ; et de 45 à 55 % en volume, de préférence environ 50 % en volume d'éthanol ; par rapport aux 100 % en volume de la composition d'électrolyte.

10. Procédé selon l'une des revendications précédentes, **caractérisé en ce que** la surface micro-structurée est revêtue avec au moins un polymère biocompatible, de préférence avec au moins un polymère bio-résorbable.

11. Procédé selon l'une des revendications précédentes, **caractérisé en ce que** l'implant est un stent.
